# EUROPEAN PATENT APPLICATION

(11) **EP 4 725 485 A1**
(43) Date of publication of application: **15.04.2026**
(21) Application number: 25205729.4
(22) Date of filing: 30.09.2025
(51) Int. Cl.: A61K 31/495, A61P 35/00, C07D 239/42

(54) **CD97 AND GSDME INTERACTION INHIBITOR HAVING ANTI-TUMOR ACTIVITY AND APPLICATION THEREOF**

(30) Priority: 11.10.2024 CN 202411413287
(71) Applicant: Jilin University, Changchun, Jilin 130000 (CN)
(72) Inventor: Wang, Yufeng, Changchun City, 130000 (CN); Li, Lingyu, Changchun City, 130000 (CN); Xiang, Jinbao, Changchun City, 130000 (CN); Liang, Chongyang, Changchun City, 130000 (CN); Wang, Ziming, Changchun City, 130000 (CN); Gao, Qiansong, Changchun City, 130000 (CN); Li, Xinze, Changchun City, 130000 (CN)
(74) Representative: Patent 42

(57) **Abstract**

The disclosure belongs to the technical field of medicine. Provided are a CD97 and GSDME interaction inhibitor having anti-tumor activity and an application thereof. A compound screened in the disclosure enhances the susceptibility of NK cells to kill tumor cells by inhibiting the interaction between GSDME and CD97 in tumor cells, so as to induce an immune anti-tumor effect. Moreover, the compound enhances the susceptibility of tumor cells to pyroptosis by blocking the protective effect of CD97 on cleavage of GSDME proteins. Therefore, the compound can be used for treating GSDME and CD97 double-positive malignant tumors, and combined chemotherapy, immunotherapy, etc., to enhance the susceptibility of malignant tumors to chemotherapy and immunotherapy, prolonging the overall survival period of patients with malignant tumors. Specifically provided is a compound having a structure set forth in formula (I), a pharmaceutically acceptable salt, a solvent compound or a deuterated compound thereof.

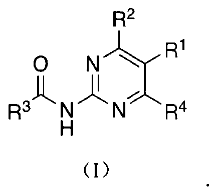

## Description

### TECHNICAL FIELD

The disclosure belongs to the technical field of medicine, and especially relates to a CD97 and GSDME interaction inhibitor having anti-tumor activity and an application thereof.

### BACKGROUND

Malignant tumor is one of the most severe diseases currently threatening human health. The data from International Agency for Research on Cancer of the World Health Organization showed that, in 2012, over 14 million new cases of malignant tumors were present worldwide, and about 8 million death cases were mainly caused by malignant tumors. Malignant tumor has become a major public health issue in China. Moreover, since 2010, it has surpassed the cardiovascular and cerebrovascular diseases to be the main cause of death in Chinese population. With the acceleration of population aging and changes in environmental and lifestyle, it is expected that in the coming decades, the number of cases and deaths from malignant tumors in China will constantly increase, posing a severe threat to the life and health of people and also bringing a heavy burden of disease to society. Currently, conventional treatment methods for malignant tumors include surgical operation treatment, radiotherapy, chemotherapy, biological modulation therapy, etc, of which, chemotherapy, as a systemic treatment approach, occupies an important position and cannot be replaced by the surgical operation and radiotherapy in the treatment of malignant tumors. In recent years, the emergence of new chemotherapeutic drugs has significantly improved the prognosis of patients with tumors. However, the toxicity of chemotherapeutic drugs and the drug resistance of tumor cells have emerged as two major problems that are difficult to be solved in chemotherapy.

Gasdermin E (abbreviated as GSDME), encoded by the *DFNA5* gene, was initially identified as a genetic cause of non-syndromic hearing loss. In recent years, GSDME is recognized as a precursor of a pore-forming protein, which can be cleaved by apoptosis-related Caspase-3 or granzyme B (GZMB) secreted by immune cells, to release the N terminus of a pore-forming domain containing an amino terminal. The N terminus binds to the membrane to form a pore with an inner diameter of 10-15 nanometers (nm), thereby triggering pyroptosis of tumor cells. The pyroptosis of tumor cells not only inhibits the growth of the tumor itself but also increases the proportion of infiltrating CD8⁺ T and natural killer (NK) cells within the tumor, thereby promoting the "cold-hot transition" of the tumor. Therefore, the tumor treatment strategy targeting GSDME is a new direction in the treatment of malignant tumors. However, malignant tumors with positive GSDME do not exhibit high susceptibility to chemotherapy and immunotherapy.

CD97 is a member of the epidermal growth factor-seven transmembrane (EGF-TM7) receptor family, and belongs to the class B G-protein-coupled receptors (GPCRs). Studies have shown that CD97 can participate in tumor differentiation, migration, invasion, and metastasis processes, which is highly expressed in many cancers, including pancreatic cancer, cervical cancer, colon cancer, thyroid cancer, oral cancer, leukemia, etc. Therefore, the disclosure provides a CD97 and GSDME interaction inhibitor having anti-tumor activity and an application thereof.

### SUMMARY

An objective of the disclosure is to provide a CD97 and GSDME interaction inhibitor having anti-tumor activity and an application thereof to solve the problems raised in the background described-above.

The objective of the disclosure is realized by the following technical solutions.

Provided is a compound, a pharmaceutically acceptable salt, a solvent compound, or a deuterated compound thereof, with a structure being set forth in formula (I): 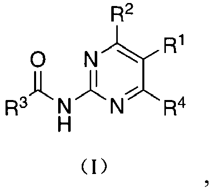 where R¹ is selected from methyl, deuterated methyl, and fluoromethyl;
R² is selected from hydrogen, deuterium, halogen, C1-C3 alkyl, and C1-C3 alkoxy;
R³ and R⁴ are independently selected from cycloalkyl, aryl, and heteroaryl, where the cycloalkyl, aryl, and heteroaryl of R³ and R⁴ are optionally substituted with 1, 2, 3, 4, or 5 R;
R is selected from hydrogen, deuterium, halogen, hydroxyl, amino, trifluoromethyl, cyano, ester, carbonyl, acyl, aminoacyl, amido, sulfonyl, aminosulfonyl, sulfonamido, alkyl, alkoxy, aryl, furyl, thienyl, pyridyl, oxazolyl, and pyrazolyl.

In an embodiment, R² is selected from hydrogen and deuterium.

In an embodiment, R³ and R⁴ are selected from aryl and heteroaryl.

An application of the compound, the pharmaceutically acceptable salt, the solvent compound, or the deuterated compound thereof as a CD97 and GSDME interaction inhibitor in the preparation of a drug for treating a cancer is provided.

In an embodiment, the cancer includes a malignant tumor with dual positive expression of GSDME and CD97, an amino acid sequence of GSDME is set forth in SEQ ID NO.1, and an amino acid sequence of CD97 is set forth in SEQ ID NO.2.

In an embodiment, the malignant tumor is lung cancer.

In an embodiment, the malignant tumor is breast cancer.

In an embodiment, the malignant tumor is colorectal cancer.

In an embodiment, the malignant tumor is melanoma.

The following terms are used herein and are defined as follows.

The term "pharmaceutically acceptable salt" refers to a salt of the compound of the disclosure, which is pharmaceutically acceptable and capable of retaining the biological effectiveness of the free base without other adverse side effects. Such a salt includes an acid addition salt formed with an inorganic acid or an organic acid. The inorganic acid includes hydrochloric acid, hydrobromic acid, sulfuric acid, nitric acid, phosphoric acid, etc.; and the organic acid includes acetic acid, propionic acid, caproic acid, cyclopentanepropionic acid, glycolic acid, pyruvic acid, trifluoroacetic acid, formic acid, lactic acid, malonic acid, succinic acid, malic acid, maleic acid, fumaric acid, tartaric acid, citric acid, benzoic acid, cinnamic acid, mandelic acid, methanesulfonic acid, ethanesulfonic acid, benzenesulfonic acid, naphthalenesulfonic acid, camphorsulfonic acid, glucoheptonic acid, gluconic acid, glutamic acid, hydroxynaphthoic acid, salicylic acid, stearic acid, muconic acid, etc. Such a salt also includes a salt formed when an acidic proton present on the parent compound is replaced by a metal ion, such as an alkali metal ion or an alkaline earth metal ion; for example, a sodium salt, a potassium salt, a calcium salt, and a magnesium salt. Such a salt also includes a coordination compound formed with an organic base. The organic base includes ethanolamine, diethanolamine, triethanolamine, N-methylglucamine, etc. The pharmaceutically acceptable salts of the disclosure can be synthesized from a parent compound containing acid radical or base by conventional chemical methods. Generally, the salt is prepared by: reacting the compounds in free acid or base form with a stoichiometric amount of the appropriate base or acid in water or an organic solvent or a mixture of both. Generally, non-aqueous media such as ether, ethyl acetate, ethanol, isopropanol, or acetonitrile are preferably selected. In addition to the salt form, the compound provided in the disclosure also exists in a prodrug form. The prodrug of the compound herein readily undergoes chemical changes under physiological conditions to convert into the compound of the disclosure. Moreover, the prodrug can be converted into the compound of the disclosure by chemical or biochemical methods within the in vivo environment.

The term "prodrug" refers to a compound that is obtained by chemically modifying the structure of the compound of the disclosure, exhibits little or no activity in vitro, and releases an active compound through enzymatic or non-enzymatic conversion in vivo to exert pharmacological effects. When specific salts, pharmaceutical compositions, compositions, excipients, etc. are "pharmaceutically acceptable", it means that the salts, pharmaceutical compositions, compositions, excipients, etc. are generally non-toxic, safe, and suitable for use in subjects, preferably mammalian subjects, and more preferably human subjects.

The term "subject" refers to any animal that, according to the embodiments of the disclosure, is about to receive or has already received administration of the compound or pharmaceutical composition, with mammals being preferred and humans being the most preferred. As used herein, the term "mammal" includes any mammal. Examples of mammals include, but are not limited to, cattle, horses, sheep, pigs, cats, dogs, mice, rats, rabbits, guinea pigs, monkeys, and humans, with humans being the most preferred.

The term "solvent compound" refers to a substance formed by combining the compound of the disclosure with a pharmaceutically acceptable solvent. The pharmaceutically acceptable solvent includes water, ethanol, acetic acid, etc. The solvent compound includes stoichiometric solvent compounds and non-stoichiometric solvent compounds, with hydrates being preferred. Certain compounds of the disclosure may exist in either non-solvated form or solvated form, including hydrate forms. Generally, the solvated form and the non-solvated form are considered equivalent and are included within the scope of the disclosure.

When referring to certain elements, such as hydrogen or H, it is intended to include all isotopes of the element. For example, if an R group is defined to include hydrogen or H, it also includes deuterium and tritium. Therefore, isotopically labeled compounds are included in the scope of the disclosure.

The terms "treatment" or "treating" refer to the improvement, prevention, or reversal of a disease or disorder, or at least one discernible symptom thereof. In another embodiment, "treatment" or "treating" refers to the improvement, prevention, or reversal of at least one measurable physiological parameter associated with the disease or disorder being treated, even if the disease or disorder itself may not have been identified in the mammal. In yet another embodiment, "treatment" or "treating" refers to slowing the progression of the disease or disorder, whether physically, such as the stabilization of discernible symptoms, or physiologically, such as the stabilization of a physiological parameter, or a combination of both. In still another embodiment, "treatment" or "treating" refers to delaying the onset of the disease or disorder. In certain embodiments, the compound of the disclosure is administered as a prophylactic measure. As used herein, "prevention" or "preventing" refers to reducing the risk of acquiring a given disease or disorder. In a preferred mode of the embodiment, the specified compound is administered as a prophylactic measure to subjects, such as subjects with a family history or predisposition to cancer or autoimmune diseases.

The term "halogen" refers to fluorine, chlorine, bromine, and iodine. The term "alkyl" refers to a straight-chain or branched saturated hydrocarbon group. In some embodiments, the alkyl is C1-C3 alkyl; and in other embodiments, the alkyl is C1-C6 alkyl. Examples of alkyl include, but are not limited to, methyl, ethyl, propyl (including isopropyl and n-propyl), butyl (including n-butyl and isobutyl), pentyl (including n-pentyl, isopentyl, and neopentyl), and hexyl. The alkyl can be monovalent (e.g., methyl), divalent (e.g., methylene), or multivalent (e.g., methine), and can be mono-substituted (e.g., -CH₂F) or poly-substituted (e.g., -CF₃).

The term "alkoxy" refers to a group with the structure -O-alkyl, where the alkyl is defined as above. Specific examples include, but are not limited to, methoxy, ethoxy, n-propoxy, isopropoxy, n-butoxy, tert-butoxy, isobutoxy, n-pentyloxy, etc.

The term "alkenyl" refers to a straight-chain or branched hydrocarbon group containing one or more carbon-carbon double bonds. In some embodiments, the alkenyl is C2-C4 alkenyl; in other embodiments, the alkenyl is C2-C6 alkenyl; and in yet other embodiments, the alkenyl is C2-C8 alkenyl.

The term "ring" refers to substituted or unsubstituted cycloalkyl, cycloalkenyl, cycloalkynyl, aryl, heterocycloalkyl, heterocycloalkenyl, heterocycloalkynyl, and aryl. The ring includes monocyclic, bicyclic, and polycyclic structures. The number of atoms in the ring is defined as the ring size.

The term "hetero" refers to a heteroatom or a heteroatomic group (a group containing heteroatoms), i.e., atoms other than carbon (C) and hydrogen (H), as well as groups containing these heteroatoms, such as oxygen (O), nitrogen (N), and sulfur (S).

The term "cycloalkyl" refers to cyclic alkyl containing monocyclic, bicyclic, or tricyclic systems, where the bicyclic or tricyclic systems include spiro ring, bridged ring, and fused ring; and the alkyl can be monovalent, divalent, multivalent, or mono-substituted or poly-substituted.

The term "cycloalkenyl" refers to cyclic alkenyl containing one or more unsaturated carbon-carbon double bonds, where the bicyclic or tricyclic systems include spiro ring, bridged ring, and fused ring; and the alkyl can be monovalent, divalent, multivalent, or mono-substituted or poly-substituted.

The term "cycloalkynyl" refers to cyclic alkynyl containing one or more carbon-carbon triple bonds, including monocyclic, bicyclic, and polycyclic systems, where the bicyclic or tricyclic systems include spiro ring, fused ring, and bridged ring. The alkynyl can be monovalent, divalent, multivalent, or can be mono-substituted or poly-substituted.

The term "heterocycloalkyl" refers to cyclized heteroalkyl, including monocyclic, bicyclic, and polycyclic systems, where the bicyclic or tricyclic systems include spiro ring, fused ring, and bridged ring.

The term "heterocycloalkenyl" refers to cyclized heteroalkenyl, including monocyclic, bicyclic, and polycyclic systems, where the bicyclic or tricyclic systems include spiro ring, fused ring, and bridged ring.

The term "heterocycloalkynyl" refers to cyclized heterocycloalkynyl, including monocyclic, bicyclic, and polycyclic systems, where the bicyclic or tricyclic systems include spiro ring, fused ring, and bridged ring.

The term "aromatic ring" or "aryl" refers to polyunsaturated carbocyclic systems, including monocyclic, bicyclic, and polycyclic systems, where at least one ring exhibits aromaticity; the polyunsaturated carbocyclic system can be monovalent, divalent, multivalent, and can also be mono-substituted or poly-substituted.

The term "substituted" means that any one or more hydrogen atoms on a specific atom are replaced by substituents, which may include variants of deuterium and hydrogen, as long as the valence of the specific atom remains normal and the substituted compound is stable. When the substituent is oxygen (i.e., =O), it means that two hydrogen atoms are replaced. Oxygen substitution does not occur on aryl. The term "optionally substituted" means that substitution may or may not occur, and unless otherwise specified, the type and number of substituents can be any as long as they are chemically feasible.

Compared with the prior art, the disclosure has the following advantageous effects.

The disclosure discloses an application of a compound having a structure set forth in formula (I), a pharmaceutically acceptable salt, a solvent compound, or a deuterated compound thereof in the preparation of a drug for treating a cancer. The compound enhances the susceptibility of NK cells to kill tumor cells by inhibiting the interaction between GSDME and CD97 in tumor cells, so as to induce an immune anti-tumor effect. Moreover, the compound enhances the susceptibility of tumor cells to pyroptosis by blocking the protective effect of CD97 on cleavage of GSDME proteins within the tumor cells. Therefore, the compound can be used for treating GSDME and CD97 double-positive malignant tumors, and combined chemotherapy, immunotherapy, etc., to enhance the susceptibility of malignant tumors to chemotherapy and immunotherapy, so as to prolong the overall survival period of patients with malignant tumors.

### BRIEF DESCRIPTION OF THE DRAWINGS

FIG. 1a shows the preparation process for two types of SERS nanoprobes and the schematic construction of a screening platform; and
FIG. 1b shows a schematic diagram of the screening process for an inhibitor using a combinatorial compound library (20 compounds at 7 µM each).
FIG. 2 shows the results of the effect of the compound on tumor cell susceptibility to the NK cell-induced killing, with data presented as mean ±SEM.
FIG. 3a is a model diagram for establishing a screening system; and
FIG. 3b shows the Elisa test result about IFN-γ release.
FIG. 4a is the western blot results showing the effect of short interfering RNA (siRNA) on ADGRE5 gene (encoding CD97 protein) expression in NCI-H446, NCI-H1688, and A375 cells;
FIG. 4b shows tumor cell lysis induced by amplified human NK cells (at an E/T ratio of 5:1) against NCI-H446, NCI-H1688, or A375 tumor cells treated with Ctrl, KD1, or KD2;
FIG. 4c shows the percentage of NK cell-induced tumor cell lysis under different treatment conditions;
FIG. 4d shows the percentage of CD107a expression on NK cell surfaces under various treatment conditions;
FIG. 4e shows microscopic images of NCI-H446 cells (Ctrl, KD1, or KD2) co-incubated with or without primary human NK cells for 16 hours; and
FIG. 4f shows primary human NK cell-induced lysis of GSDME^{-/-} or GSDME^{+/+} tumor cells under CD97 knockdown (KD) or Ctrl conditions, where all data are presented as mean ±SEM, and one-way analysis of variance was performed in FIGS. 4d-4h, Ns, p > 0.05; *, p < 0.05; **, p < 0.01; ***, p < 0.001; ****, p < 0.0001.
FIG. 5a shows the in vivo growth curve of B16 tumors;
FIG. 5b shows the in vivo growth curve of CT26 tumors; and
FIG. 5c shows the western blot analysis demonstrating ectopic expression of mouse GSDME (mGSDME) in 4T1 cells with low endogenous GSDME expression (left panel); and shows tumor growth of empty vector (EV)- or mGSDME-transfected 4T1 cells in BALB/c mice treated with Chol-scramble or Chol-siCD97 (right panel, n = 7 mice per group).
FIG. 6a shows the results of NCI-H446 cells treated with scramble siRNA (Ctrl), CD97-specific siRNA1 (KD1), or CD97-specific siRNA2 (KD2), followed by stimulation with or without primary human NK cells for 16 hours (left panel); and shows quantitative determination of the NT/FL ratio (right panel);
FIG. 6b shows the cleavage of GSDME using recombinant Caspase-3 (rCASP3); and shows quantification of the NT/FL ratio (right panel);
FIG. 6c shows the western blot analysis of GSDME;
FIG. 6d shows tumor cell lysis of NCI-H446 cells induced by primary human NK cells under CD97 knockdown (KD) or Ctrl conditions, in the presence of DMSO, zDEVD (100 µM, pre-treatment of tumor cells for 1 hour), or a GZMB inhibitor (100 µM, pre-treatment of NK cells for 1 hour);
FIG. 6e shows pull-down of NCI-H446 tumor cell lysates using IgG or anti-GSDME antibody;
FIG. 6f shows mixing of CD97-his (rCD97-his) and GAPDH (rGAPDH), followed by pull-down using IgG or anti-his antibody;
FIG. 6g shows the design of different deletion mutants centered around the cleavage site of GSDME according to different domains of GSDME;
FIG. 6h evaluates the ability of GSDME deletion mutants to interact with GSDME via IP (left panel) and quantifies their binding affinity by measuring the amount of bound GSDME (right panel); and
FIG. 6i shows western blot analysis of recombinant GSDME (rGSDME) incubated in vitro with rCASP3 or recombinant granzyme B (rGZMB) for 1 hour, where 1.25 µg of rGSDME was incubated with indicated doses of rCASP3 (0, 3.125, 6.25, 12.5, 25, 50 × 10⁻² IU) (left panel) or rGZMB (0, 0.75, 1.5, 3, 6 ng/µL) (right panel) in the presence of 2.5 µg rCD97.
FIG. 7a shows the ultraviolet-visible absorption spectrum of silver nanoparticles (AgNPs) (diluted threefold); and
FIG. 7b shows the transmission electron microscopy (TEM) image of AgNPs.
FIG. 8 shows the electron microscopy characterization image of silver-magnetic nanoparticle complex in Embodiment 5.
FIG. 9a shows the screening results obtained by surface-enhanced Raman spectroscopy (SERS) in Embodiment 5; and
FIG. 9b shows a bar chart of the intensities of the characteristic SERS peak at 1587 cm⁻¹ across different small-molecule identifiers in Embodiment 5.
FIG. 10a shows the SERS detection results following treatment with gradient concentrations of WXLL-5471 in Embodiment 5; and
FIG. 10b shows a scatter plot illustrating the intensities corresponding to the characteristic peak at 1587 cm⁻¹ in Embodiment 5.
FIG. 11a shows immunoprecipitation analysis of lysates from 293T cells expressing Flag-GSDME, treated with WXLL-5471 (0.5, 1, 5 µM) for 18 hours, using an anti-Flag antibody;
FIG. 11b shows tumor cell lysis of NCI-H446 cells induced by primary human NK cells (at an E/T ratio of 5:1 for 16 hours; N = 5 donors per group) following pre-treatment with DMSO or WXLL-5471 (5 µM) for 18 hours;
FIG. 11c shows western blot analysis of NCI-H446 cells pre-treated with DMSO or WXLL-5471 (5 µM) for 18 hours, followed by stimulation with or without primary human NK cells for 16 hours (left panel); and shows quantitative determination of the NT/FL ratio (right panel).
FIG. 12a shows the body weight changes of mice treated with the compound and that of mice in control groups during the treatment period;
FIG. 12b shows the blood routine examination results of mice treated with the compound and mice in the control group over 7 consecutive days of treatment;
FIG. 12c shows the biochemical parameter results of mice treated with the compound and mice in the control group over 7 consecutive days of treatment, where all data are expressed as mean ±SEM, and statistical analysis is performed using an unpaired t-test; Ns, p > 0.05; *, p < 0.05.
FIG. 13a shows the growth of CT26 and B16 tumors in BALB/c and C57BL/6 mice following intraperitoneal administration of saline or 100 mg/kg WXLL-5471 (8 mice per group);
FIG. 13b shows the growth of mGSDME^{wT}, mGSDME^{D267A/D270A}, and mGSDME^{-/-} B16 tumors in C57BL/6 mice treated with saline or 100 mg/kg WXLL-5471 (5 mice per group);
FIG. 13c presents mass cytometry-based T-sne plot depicting intratumoral immune cell composition in B16 tumors after 7 consecutive days of treatment with saline or WXLL-5471 (n = 5 mice per group);
FIG. 13d is a volcano plot showing fold line changes and adjusted p-values for immune cell type;
FIG. 13e is a radar chart illustrating expression levels of GZMB, CD69, Ki-67, IFN-γ, CD107a, and PD1 in CD8⁺ T cells (left panel) and TIL-NK cells (right panel) from B16 tumors treated with or without WXLL-5471;
FIG. 13f shows comprehensive immune scores for the six markers in each group of FIG. 13e;
FIG. 13g shows PD1 expression in TIL-NK and CD8⁺ T cells within B16 tumors;
FIG. 13h shows tumor volume in B16 tumor-bearing mice treated with combination therapy (WXLL-5471 + anti-PD-1) or monotherapy (WXLL-5471 or anti-PD-1 alone) (n = 10 per group); and
FIG. 13i shows survival rates in B16 tumor-bearing mice treated with combination therapy (WXLL-5471 + anti-PD-1) or monotherapy (WXLL-5471 or anti-PD-1 alone) (n = 10 per group); Ns, p > 0.05; *, p < 0.05; **, p < 0.01; ***, p < 0.001; ****, p < 0.0001.

### DETAILED DESCRIPTION

In order to have a clearer understanding of the technical features, objective and beneficial effects of the disclosure, the technical solutions of the disclosure are described in detail below, but it is not to be understood as a limitation of the implementable scope of the disclosure. Unless otherwise specified, the experimental methods in the embodiments mentioned below are conventional, and the reagents and materials are commercially available.

### Embodiment 1: screening of CD97 molecule using horizon G-PCR siRNA library

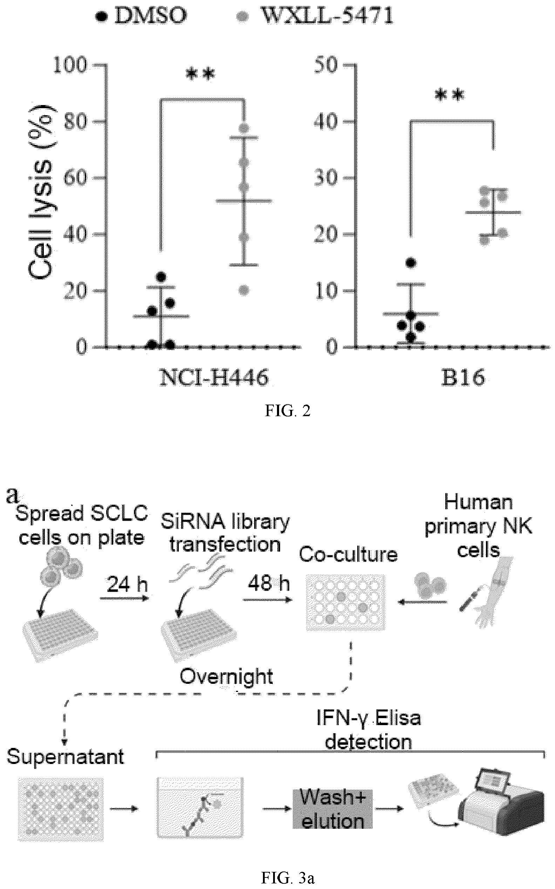

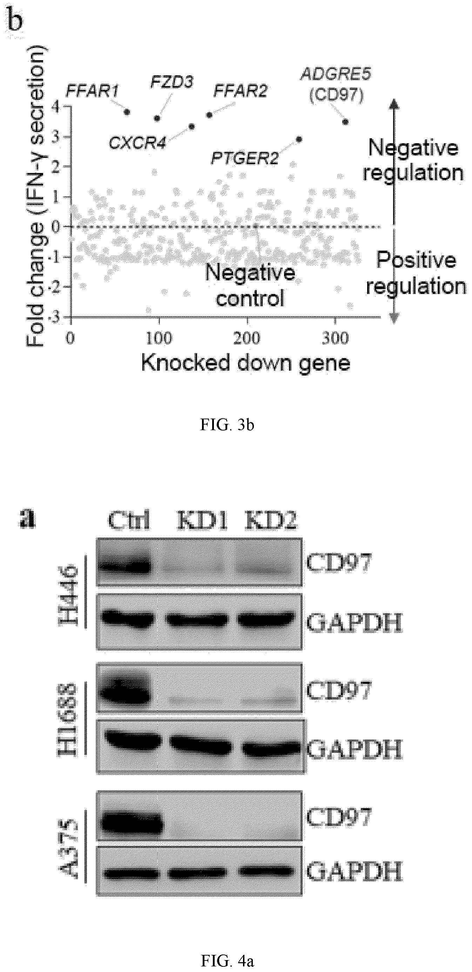

As shown in FIG. 3a, a screening system was first constructed: GSDME positive cell line NCI-H446 was spread in a 96-well plate, and transfected with a horizon G-PCR siRNA library (containing 396 candidate genes) after 24 hours; after 48 hours of transfection, Naive NK cells were added at an E/T ratio of 5:1; the system was co-incubated overnight, and supernatant was collected for ELISA analysis to detect the release of IFN-γ, thereby indirectly reflecting the susceptibility of tumor cells to the NK cell-induced killing through the extent of NK cell activation. Using the screening system, genes *FFAR1, FFAR2, FZD3, CXCR4, PTGER2,* and *ADGRE5* with significantly upregulated IFN-γ release after knockdown were screened. On this basis, NK cell-induced killing experimental verification was further conducted, and it was eventually found that the knockdown of *ADGRE5,* that is, the CD97 protein, could significantly enhance the susceptibility of small cell lung cancer (SCLC) cells to the NK cell-induced killing (FIG. 3b).

### Embodiment 2: enhancement of the susceptibility of GSDME-positive tumor cells to immune killing by knockdown of CD97

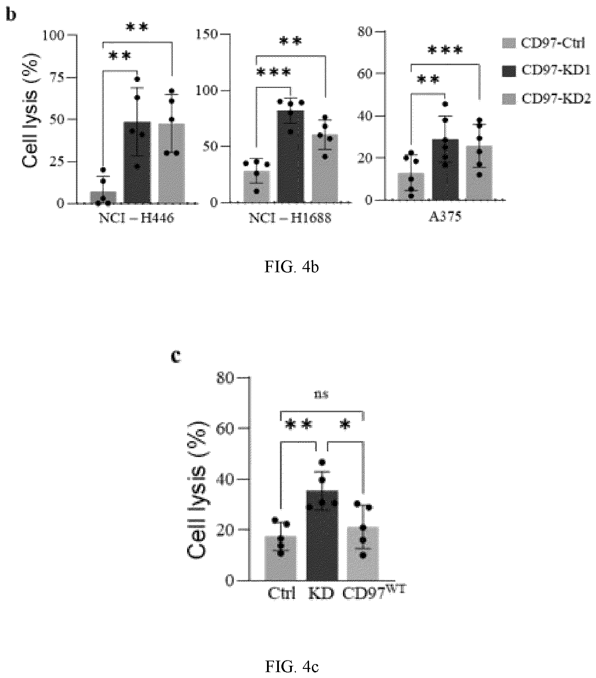

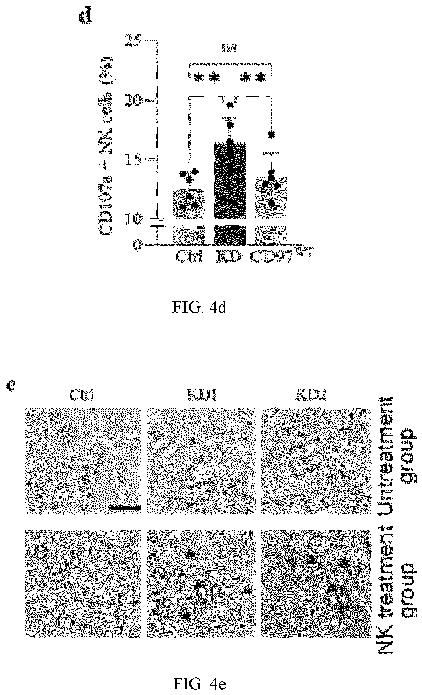

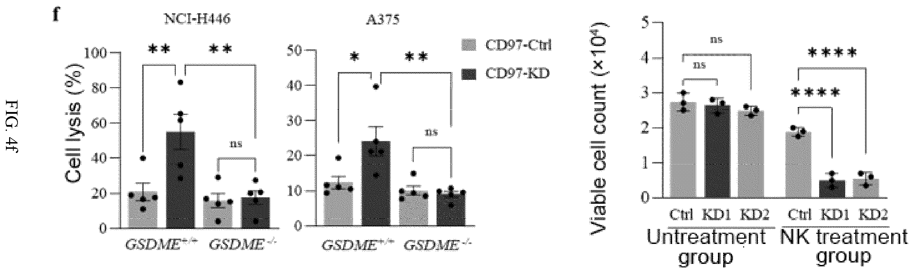

Using siRNA, CD97 (CD97-KD) was knocked out in three GSDME-expressing tumor cell lines: NCI-H446, NCI-H1688, and A375 (FIG. 4a). After incubating the transfected cells and the donor-amplified NK cells for 4 hours, the susceptibility of tumor cells to the NK cell-induced killing was evaluated via the release of calcein. In each case, the knockdown of CD97 made the tumor cells more vulnerable to the NK cell-induced cytotoxicity (FIG. 4b). Conversely, in NCI-H446 tumor cells, the ectopic expression of CD97 restored their susceptibility to the CD97-KD-induced NK cell cytotoxicity (FIGS. 4c and 4d). A large number of pyroptotic bodies were observed in CD97-KD tumor cells co-incubated with NK cells (FIG. 4e). To determine whether the NK cell-induced killing is mediated by the GSDME-mediated pyroptosis when the expression of CD97 was reduced, NCI-H446 and A375 cell models lacking GSDME were constructed, and it was found that NK cells exhibited high cytotoxicity against GSDME^{+/+} tumor cells lacking CD97, but had no toxicity towards GSDME^{-/-} tumor cells (FIG. 4f). These findings indicated that the knockdown of CD97 made tumor cells sensitive to NK cell-induced killing through a mechanism that required GSDME.

### Embodiment 3: control of the in vivo growth of malignant tumors by treatment with cholesterol-modified CD97 siRNA

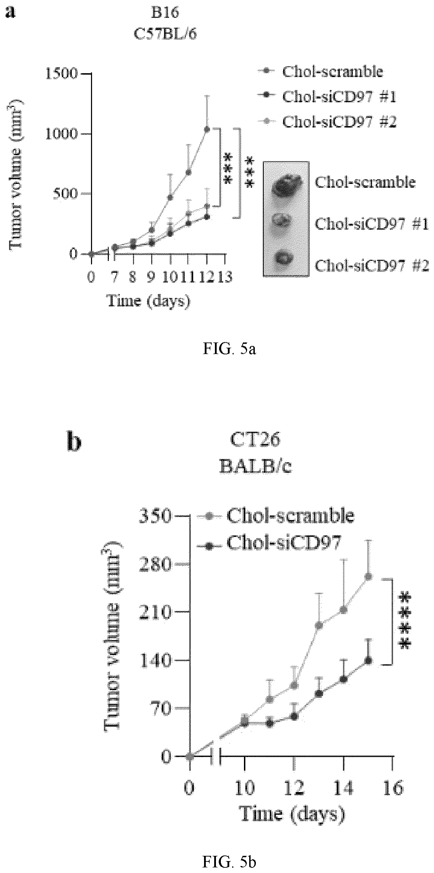

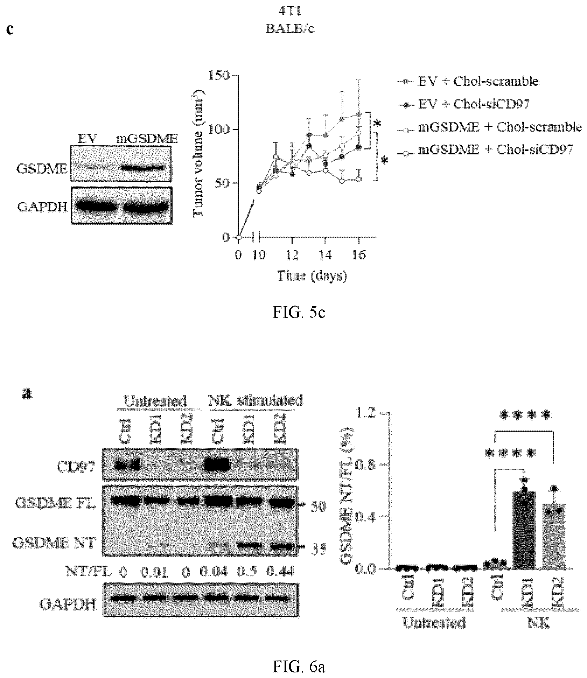

C57BL/6 mice were subcutaneously injected with 2 × 10⁵ B16F0 cells. When the subcutaneous tumors grew to a size of 5 × 5 mm, intratumoral injection of the cholesterol-modified CD97 siRNA (Chol-siCD97) or an unrelated sequence siRNA (Chol-scramble) was performed for treatment, and the growth of subcutaneous tumors in mice was recorded (FIG. 5a). Subsequently, using CT26 and 4T1 mouse tumor models (FIGS. 5b and 5c), tumor growth was recorded after the above-mentioned treatment. The experiment proved that the treatment using the cholesterol-modified CD97 siRNA could control the growth of malignant tumor in vivo.

### Embodiment 4: binding of CD97 to cleavage site of GSDME to protect GSDME from Caspase-3 and GZMB-mediated cleavage

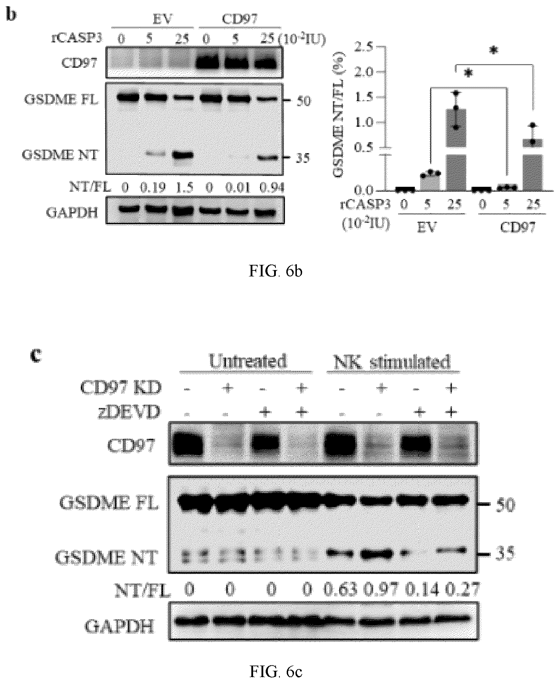

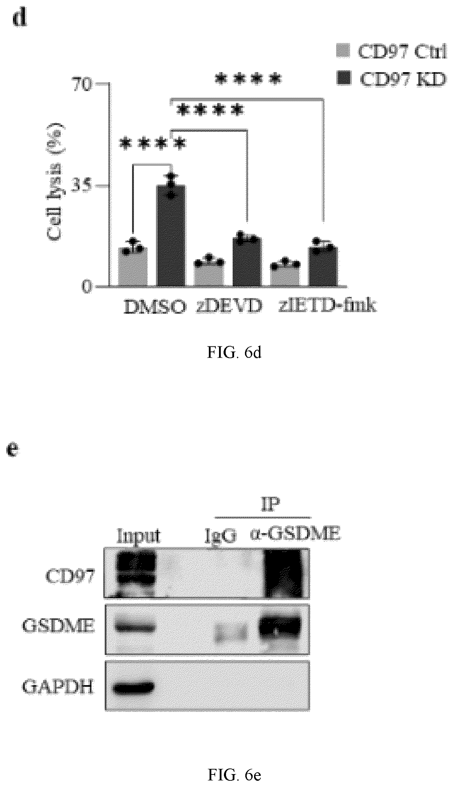

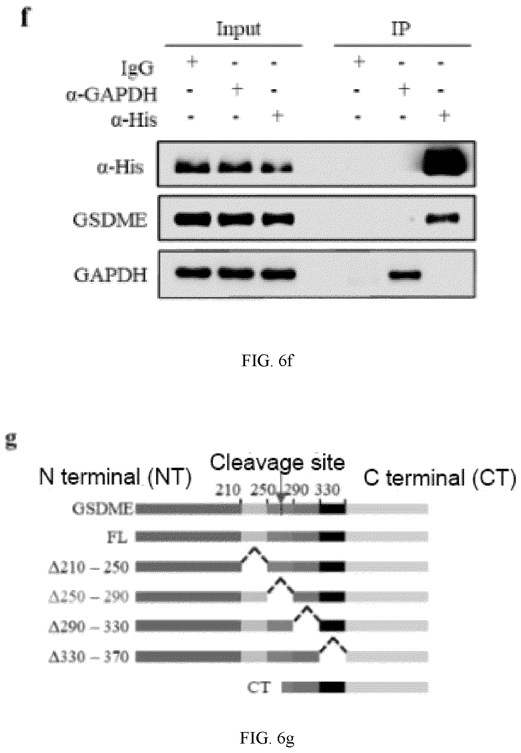

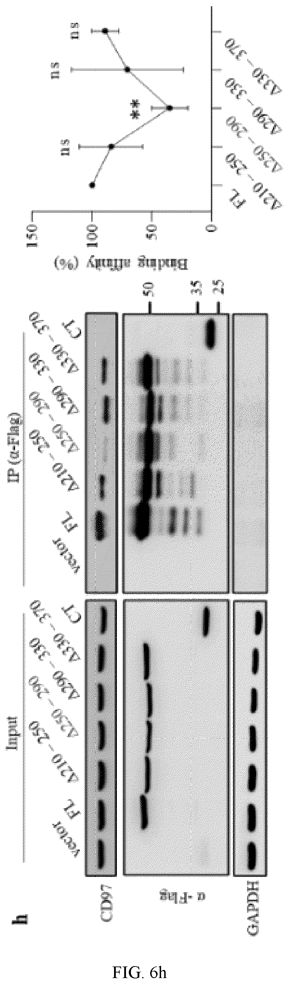

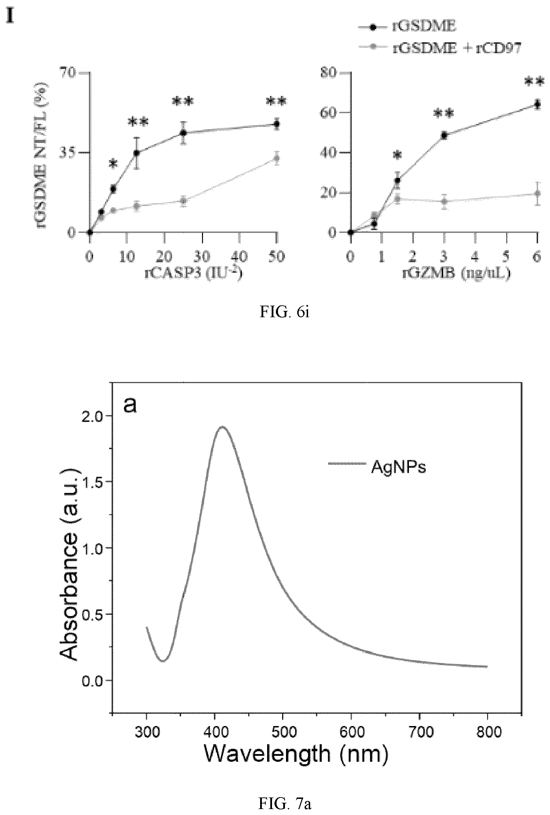

Stimulation was performed using human primary NK cells, and western blot experiment was conducted to detect the cleavage of GSDME, finding that GSDME protein was more prone to be cleaved after CD97 was knocked down in malignant cells (FIG. 6a). After overexpression of CD97, a whole-cell lysate from GSDME-positive tumor cells was treated with purified recombinant Caspase-3 protein at 37°C for 30 minutes, and western blot was used to detect the cleavage of GSDME, finding that the cleavage rate of GSDME was significantly reduced in CD97-overexpressing malignant tumor cells (FIG. 6b). The effect on the cleavage activity was rescued by pre-treating tumor cells with the Caspase-3 inhibitor zDEVD for 30 minutes (FIG. 6c) and also was rescued by pre-treating NK cells with GZMB inhibitor zIETD-fmk for 30 minutes (FIG. 6d). Further experiments using endogenous co-immunoprecipitation (FIG. 6e) and in vitro direct interaction between purified proteins (FIG. 6f) revealed that there was direct interaction between CD97 protein and GSDME protein. On the basis of different domains of GSDME, deletion mutants centered around the cleavage site of GSDME were designed (FIG. 6g). After the mutants were transfected into 293T cells, the pull-down status of the interacting protein CD97 was detected by co-immunoprecipitation technology, finding that the mutant lacking amino acids 250-290 (Δ250-290) at the cleavage site of GSDME affected CD97 binding, indicating that the binding site between CD97 and GSDME was located in the cleavage site region of GSDME (FIG. 6h). Furthermore, in vitro cleavage experiment was used to incorporate CD97 protein into the recombinant Caspase-3 and or GZMB cleavage system, and the cleavage efficiency on GSDME was significantly reduced (FIG. 6i).

### Embodiment 5: screening inhibitors of the CD97-GSDME interaction inhibitor using SERS

### 1. Preparation of silver nanoparticles (AgNPs)

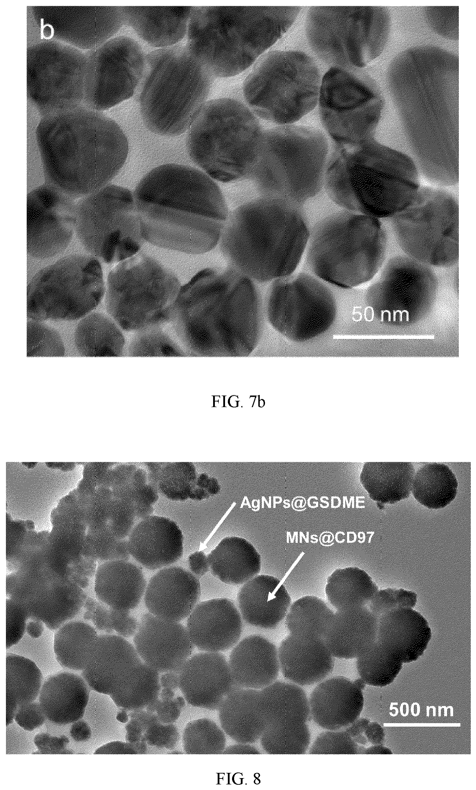

A 250 mL three-necked flask was soaked overnight in an acid bath, rinsed thoroughly the following day, and washed three times with purified water to ensure a clean, impurity-free interior. The flask was then oven-dried and cooled to room temperature. During this period, 0.018 g of AgNO₃ powder was accurately weighed and carefully transferred into the flask. Subsequently, 100 mL of ultrapure water was added, and the mixture was stirred evenly. A condenser tube was attached, and the temperature of a heating mantle was set to 100°C. The solution was heated to gentle boiling under stirring, and then 2 mL of 1% sodium citrate solution was added rapidly. The color changes of the solution were observed: from light yellow to deep yellow to grayish-green. Once the color stabilized, the temperature was slightly reduced to 90°C, and magnetic stirring was maintained for 40 minutes to obtain a silver nanoparticle dispersion system. The absorbance curve of silver nanoparticles was measured using an ultraviolet spectrophotometer: after 3-fold dilution of the silver nanoparticles, it was observed that there was a correct maximum absorption wavelength range (420-480 nm) without any other extraneous peaks (as shown in FIG. 7a), and it could be preliminarily determined that the synthesized particles had uniform sizes. The TEM image (FIG. 7b) showed that the particle size was approximately 50 nm, with relatively uniform dimensions and good dispersion. The concentration of silver nanoparticles was calculated using the formula: A = kbc (where A = Aₘₐₓ, k = 3 × 10¹¹ M⁻¹cm⁻¹, and b = 1 cm). The sample was stored at 4°C for later use.

### 2. Preparation of magnetic nanoparticles

Commercially available carboxyl magnetic beads (500 nm) from Aladdin (Shanghai, China) were prepared at a concentration of 0.5 mg/mL. The magnetic beads were washed with storage buffer and were subjected to magnetic separation three times, followed by resuspension in the storage buffer to prevent aggregation. The finished products were stored at 4°C for later use.

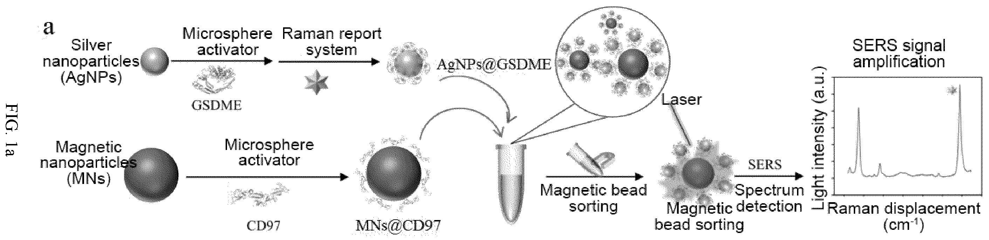

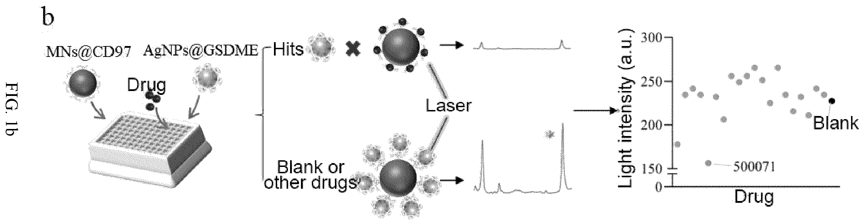

### 3. Synthesis of two types of Raman probes for screening inhibitors of the CD97-GSDME interaction; the workflow is shown in FIG. 1

(1) Preparation of AgNPs@GSDME silver probe: 1 mL of silver nanoparticles (AgNPs, 0.25 nM) was mixed with 20 µL of Tween 20 in a 1.5 mL Eppendorf (EP) tube, for uniform mixing for 20 minutes to prevent aggregation during surface modification of AgNPs. 10 µL of 1-(3-dimethylaminopropyl)-3-ethylcarbodiimide hydrochloride (EDC, 2.5 mM) and 10 µL of N-hydroxysuccinimide (NHS, 2.5 mM) were added, followed by reaction for 1 hour. The mixture was centrifuged at 4000 g for 7 minutes to remove excess activators and resuspended. Then, 2 µL of GSDME (1 mg/mL) and 25 µL of 4-MBA (1 mM; if using a 96-well plate, 4-MBA was not added at this step) were added to the solution, which was reacted at room temperature for 1 hour. The unreacted molecules were removed by centrifugation (4000 g, 7 min) and then resuspension was performed. The prepared AgNPs@GSDME was stored at 4°C for later use.
(2) Preparation of MNs@CD97 magnetic probe: 500 µL of carboxyl magnetic beads (0.5 mg/mL) was mixed with 5 µL of Tween 20, for uniform mixing for 30 minutes. Then, 10 µL of 100 mM EDC/NHS was added for activation for 1 hour. Excess activators were removed via magnetic separation, and the beads were washed with phosphate buffered saline (PBS) and resuspended. 2 µL of CD97 (1 mg/mL) was added to the magnetic bead solution for reaction at room temperature for 1 hour. The beads were magnetically separated and washed three times to remove non-specifically bound proteins, then resuspended in PBS to obtain MNs@CD97, which was stored at 4°C for later use.

### 4. Construction of CD97-GSDME interaction inhibitor screening platform

A high-throughput targeted drug screening platform (SERScreen) based on SERS was established to screen inhibitors of the CD97-GSDME interaction.

Negative control: immunoaffinity structure evaluation: Raman probes were freshly prepared by mixing a silver probe with a magnetic probe at a 3:1 volume ratio, followed by incubation for 60 minutes. Samples were immediately collected for Raman signal detection and plotting, with this signal serving as the negative control. An electron microscopy image (FIG. 8) revealed that after three times of incubation and washing, numerous silver probes (small spheres) adhered to the surface of magnetic probes (large spheres), proving successful probe preparation. Under protein-protein interaction between the silver probe and the magnetic probe, a silver-magnetic probe complex was formed. Driven by a magnetic field, magnetic aggregation was performed, and Raman detection was performed at the probe aggregation site in a standard polymerase chain reaction (PCR) tube (with 1 second exposure time, 10 accumulations averaged), and the enhanced characteristic peaks of the 4-MBA reporter could be detected.

### 5. High-throughput screening of small-molecule combinatorial compound library against the CD97-GSDME interaction target using SERScreen

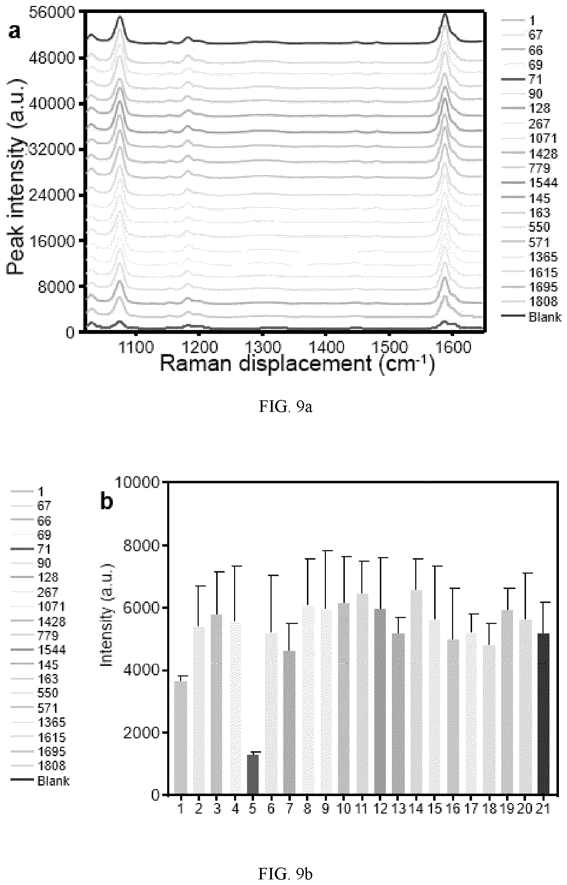

The high-throughput screening was performed in a 96-well plate: each well was added with 150 µL of AgNPs@GSDME (unlabeled with 4-MBA) and different small-molecule compounds (7 µM). Then, 50 µL of MNs@CD97 was added to each well, followed by reaction for 1 hour. Magnetic separation was performed, followed by washing three times, and resuspension was performed in PBS. Each well was added with 4 µL of 4-MBA (1 mM) for reaction for 1 hour, followed by magnetic separation, washing three times, and reaggregation. SERS detection was performed at the probe aggregation site (633 nm, 1 second of exposure time, 10 accumulations averaged). During data processing, each small molecule was detected in three batches, with at least three parallel measurements per batch. After removing impurity peaks and reducing baseline using NGSLabSpec software, final spectral data for each well were processed and plotted (FIGS. 9a-9b).

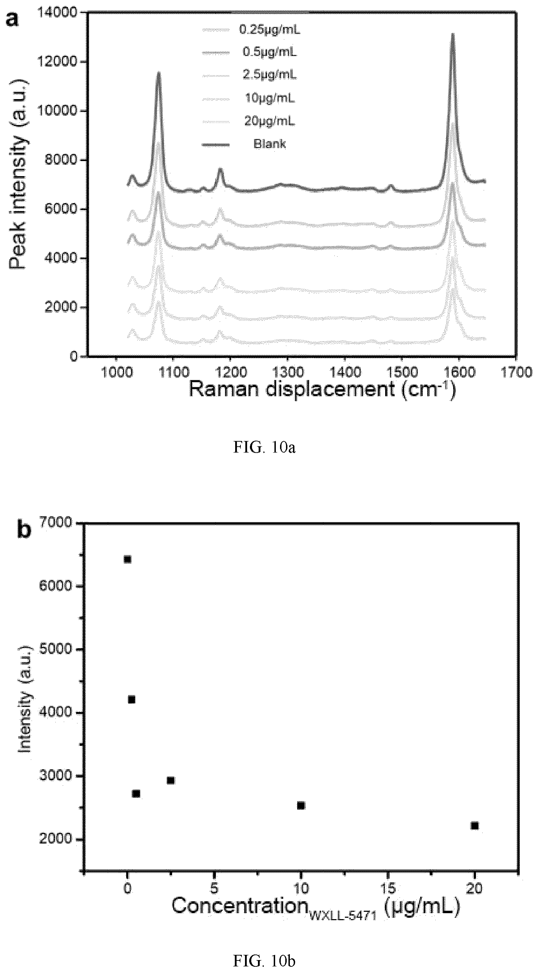

The detailed procedures are as follows:
(1) Negative control: immunoaffinity structure evaluation: Raman probes were freshly prepared by mixing a silver probe and a magnetic probe at a 150 µL:50 µL volume ratio in a 96-well plate, followed by incubation for 60 minutes. Samples were collected for immediate Raman signal detection.
(2) Experimental group: immunoaffinity inhibition efficacy evaluation: 150 µL of silver probes and 50 µL of magnetic probes were accurately pipetted, and were simultaneously added to a compound for screening; after incubation for 60 minutes, Raman signal detection was performed in a 96-well plate (with 1 second of exposure time and 10 accumulations averaged) to assess whether the compound had inhibitory binding effects. If the Raman signals became weak, the compound was possible to be an inhibitor. Potent small molecules were selected from the library for following experiments, and new small-molecule inhibitors were ultimately screened. Each group was added with a candidate small-molecule compound at a final concentration of 7 µM. The final groupings and numbering were as follows (1:1, 2:67, 3:66, 4:69, 5:71, 6:90, 7:128, 8:267, 9:1071, 10:1428, 11:779, 12:1544, 13:145, 14:163, 15:550, 16:571, 17:1365, 18:1615, 19:1695, 20:1808, 21: negative control: immunoaffinity structure). After 60 minutes of incubation with probes, Raman detection was performed. The number of small molecules causing significant reductions in Raman signals was recorded. It could be seen from the screening results that the small molecule numbered as 71 (WXLL-5471) exhibited a better inhibitory effect (FIGS. 9a and 9b), and was a potent inhibitor against the CD97-GSDME interaction target.
(3) Quantitative detection of candidate small molecule WXLL-5471: a quantitative experiment was performed on the screened inhibitor WXLL-5471. The inhibitor was diluted to a corresponding concentration gradient using buffer (FIG. 10a). Parallel Raman detection was conducted using the same method as above, and a concentration gradient scatter plot was plotted (FIG. 10b). Evaluation revealed that the SERS signals were regularly reduced with increasing small-molecule concentration, proving that the inhibitory effect of the small molecule on the CD97-GSDME interaction target was gradually increased, and the small molecule was proved to be a potent inhibitor.

Embodiment 6: the compound WXLL-5471 can enhance the cleavage of GSDME protein in tumor cells during the immune killing process

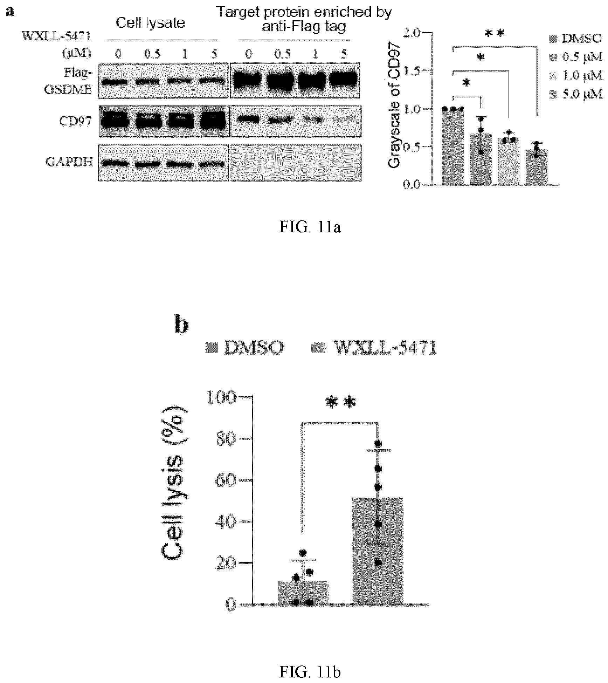

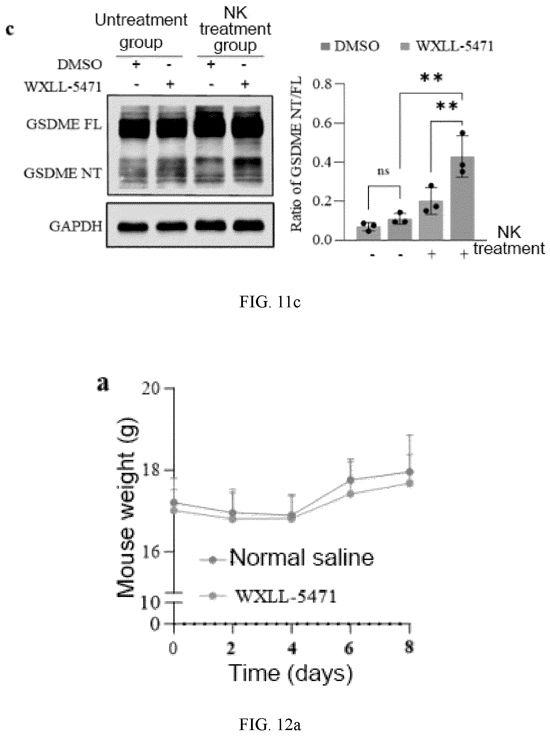

Flag-GSDME was transfected into 293T cells. After pretreatment with compound WXLL-5471 at different concentrations for 18 hours, Flag magnetic beads were used for pull-down enrichment. Finally, western blot was applied to detect the enrichment of CD97 protein. The results showed that the higher the concentration of compound WXLL-5471, the lower the enrichment efficiency of CD97 (FIG. 11a), indicating that compound WXLL-5471 had a significant inhibitory effect on the interaction between CD97 and GSDME. GSDME-positive malignant tumor cells were pretreated with 5 µM of compound WXLL-5471 or DMSO for 18 hours, followed by staining with calcein. Human primary NK cells were then added at an E/T ratio of 5:1. After co-incubation for 16 hours, the release level of calcein was detected (FIG. 11b). Additionally, after treatment under the aforementioned conditions, malignant tumor cells were sorted out for further western blot analysis to detect the cleavage of GSDME. The results demonstrated that after treatment with compound WXLL-5471, the cleavage of GSDME in malignant tumor cells was significantly increased under the immune stimulation of NK cells (FIG. 11c).

### Embodiment 7: confirmation of the safety of the compound WXLL-5471 using in vivo experiment

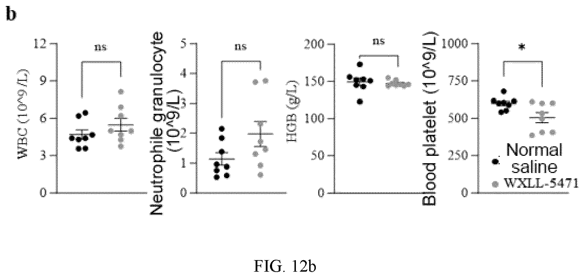

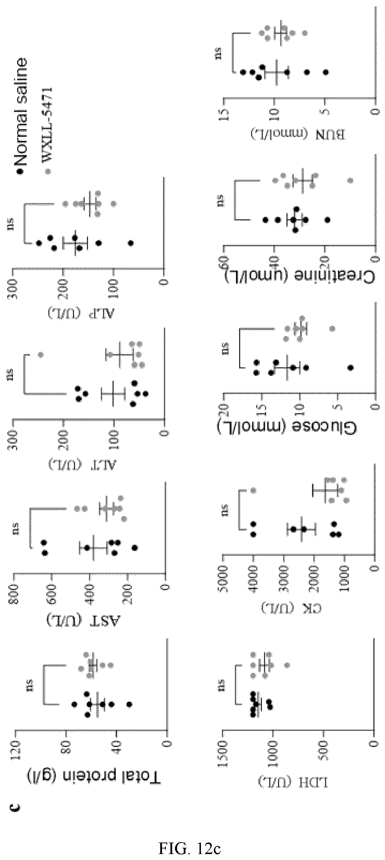

Body weight of C57BL/6 mice was recorded from 1 day before intraperitoneal injection of normal saline or WXLL-5471, which was designated as day 0. Subsequently, injection was performed and body weight was monitored for 8 consecutive days (10 mice per group), with the changes in body weight shown in FIG. 12a. After B16 tumor-bearing mice were intraperitoneally injected with normal saline or WXLL-5471 for 8 consecutive days, orbital blood samples were collected to evaluate the blood routine examination and biochemical parameters of the mice (8 mice per group for blood routine examination, and 7 mice per group for biochemical parameter detection), with the results of blood routine examination and biochemical parameters shown in FIGS. 12b and 12c, respectively. As indicated by the results in the figures, there were no significant body weight loss, hematological toxicity, and damage to liver, kidney, and myocardial functions.

### Embodiment 8: confirmation of significantly enhanced immunotherapeutic killing susceptibility of malignant tumors by the compound WXLL-5471 using in vitro experiment

NCI-H446 (FIG. 2a) and B16 (FIG. 2b) were used to pretreat human primary NK cells and mouse splenic NK cells with DMSO or WXLL-5471 (5.0 µM), respectively, to induce cell lysis for 18 hours (the E/T ratio was 5:1 for human NK cells and 20:1 for mouse NK cells; N=5 donors/mice per group). In vitro killing experiment verified that compound WXLL-5471 could significantly enhance the immune killing susceptibility of malignant tumor cells (FIGS. 2a-2b).

### Embodiment 9: the therapeutic effect of compound WXLL-5471 on malignant tumors in vivo

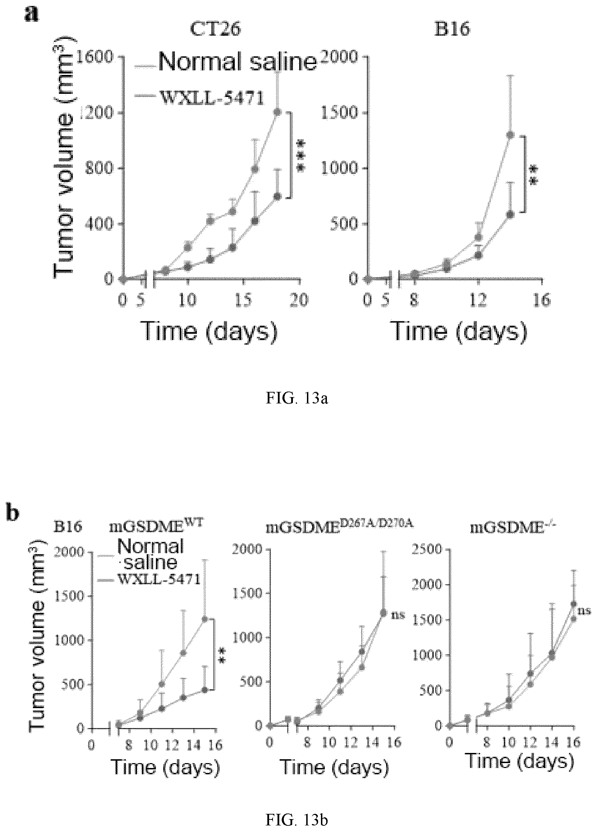

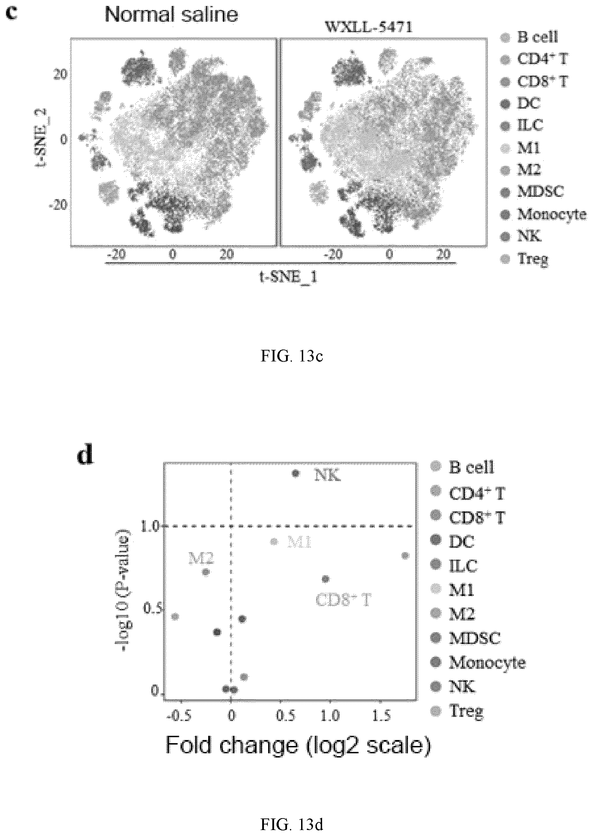

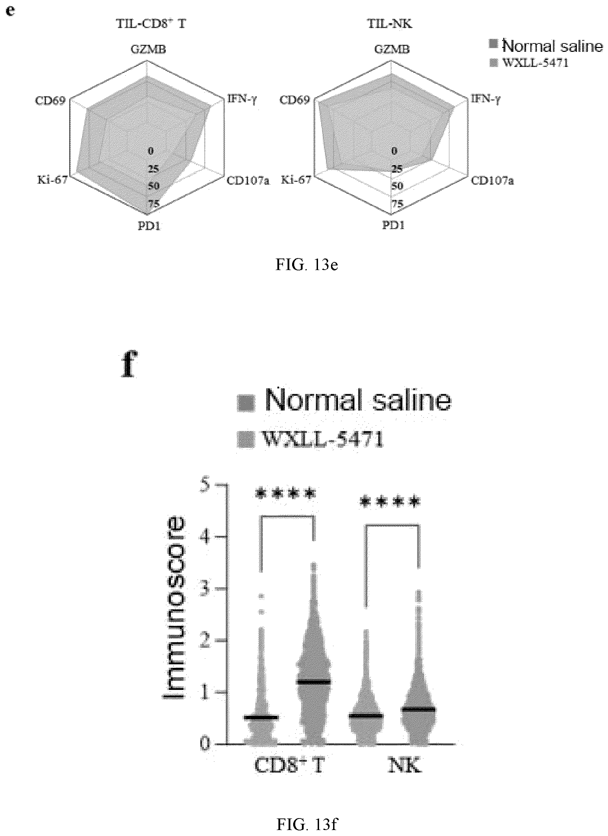

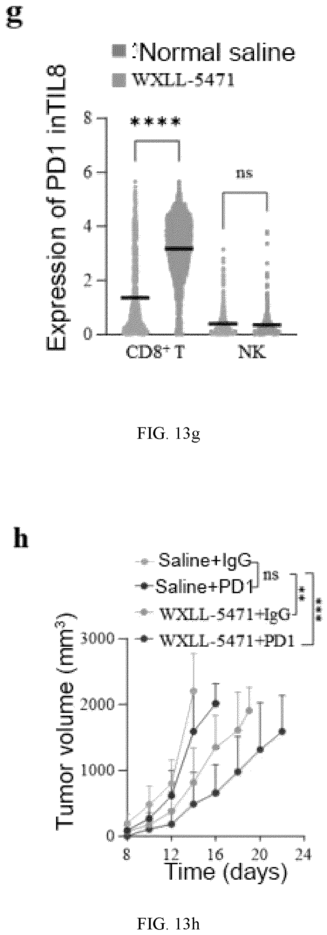

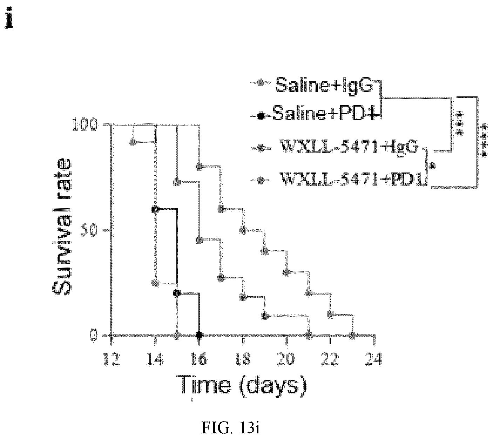

To evaluate the in vivo therapeutic effect of WXLL-5471, BALB/c mice bearing CT26 tumors and C57 mice bearing B16 tumors were intraperitoneally injected with WXLL-5471 at a dose of 100 mg/kg. On day 4 after subcutaneous injection of tumor cells, the mice were intraperitoneally injected with WXLL-5471 or normal saline daily to monitor the growth of subcutaneous tumors. The results showed that WXLL-5471 had a significant inhibitory effect on tumor growth (FIG. 13a). However, this therapeutic effect was absent in mGSDME^{D267A/D270A} and mGSDME^{-/-} B16 tumors (FIG. 13b), indicating that the in vivo anti-tumor effect of WXLL-5471 depended on the cleavage activity of GSDME in tumors. After 7 consecutive days of administration of WXLL-5471 or normal saline, mass cytometry was used to compare the differences in the number and activity of individual immune cells in the B16 tumor microenvironment (n=4 mice per group). The mass cytometry t-SNE plot depicted the composition of intratumoral immune cells in B16 tumors treated with normal saline or WXLL-5471 for 7 days (n=5 mice per group) (FIG. 13c), and the volcano plot showed the fold changes of immune cell types and adjusted p-values (FIG. 13d). It could be seen from the results of the figures that WXLL-5471 treatment led to a significant increase in the number of infiltrating NK cells (FIGS. 13c and 13d), and simultaneously significantly upregulated the activity of infiltrating NK and CD8⁺ T cells, with increased expression of CD69, GZMB, CD107a, IFN-γ, and Ki-67 (FIGS. 13e and 13f). PD1/PDL1 immune checkpoint therapy was a key therapeutic approach for the treatment of various malignant tumors such as melanoma. Exploring effective combination therapy strategies was expected to improve the clinical efficacy of malignant tumor immunotherapy. Therefore, the effect of WXLL-5471 treatment on PD1 expression in B16 tumor-infiltrating immune cells was initially investigated. WXLL-5471 treatment significantly increased PD1 expression in CD8⁺ T cells (FIG. 13g). On this basis, WXLL-5471 combined with anti-PD1 antibody more effectively controlled the growth of B16 tumors in mice (FIG. 13h) and significantly prolonged the overall survival of mice (FIG. 13i).

### Embodiment 10: synthesis of new pyrimidine derivatives

(A) Synthetic route I for aromatic amide-substituted pyrimidine biphenyl compound 5:

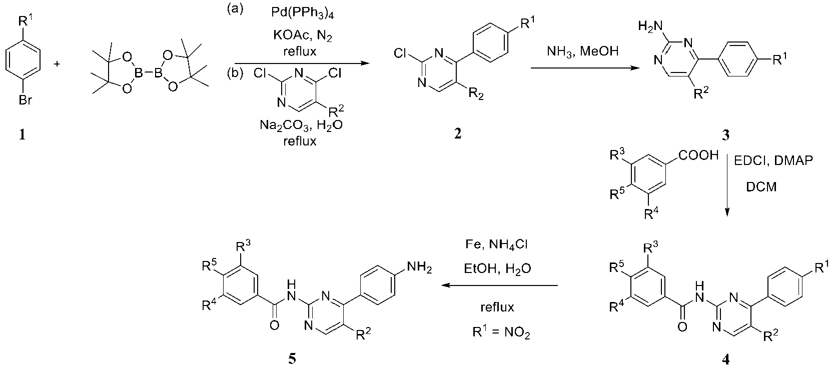

Synthetic route II of the aromatic amide-substituted pyrimidine biphenyl compound 5:

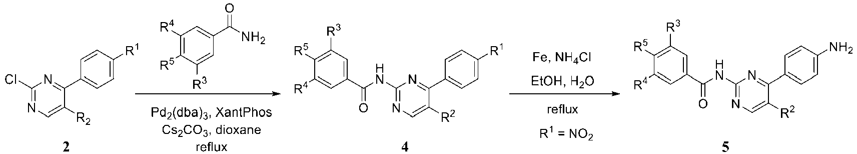

In the synthetic routes, the synthesis process for compound 2 is as follows. Bis(pinacolato)diboron (7.626 g, 30 mmol) and 1,4-dioxane (40 mL) were added in a 250 mL three-necked flask. Under nitrogen protection, tetrakis(triphenylphosphine)palladium (1.156 g, 1 mmol), potassium acetate (5.888 g, 60 mmol), and p-bromobenzene 1 (20 mmol) were added. The mixture was stirred and heated under reflux. After 8 hours, the reaction was monitored. After the raw materials were reacted completely, the reaction solution was cooled to room temperature. Under nitrogen protection, 2,4-dichloro-5-methylpyrimidine or 2,4-dichloropyrimidine (30 mmol) and a saturated sodium carbonate solution (24 mL) were added. The mixture was stirred and under reflux overnight. After an intermediate product was reacted completely by monitoring, water (100 mL) was added, and extraction was performed using ethyl acetate (3 × 50 mL). Organic phases were combined, and the combined organic phase was dried with anhydrous sodium sulfate, and a solvent was evaporated to dryness. Column chromatography separation [V (petroleum ether):V (ethyl acetate) = 10:1-4:1] and elution were performed, followed by recrystallization [V (petroleum ether):V (ethyl acetate) = 3:1] to obtain target product 2.

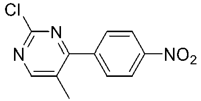

The above formula is 2-chloro-5-methyl-4-(4-nitrophenyl)pyrimidine 2.1: mp: 118-120°C; 73%; ¹H NMR (300 MHz, CDCl₃) δ 8.60 (d, *J* = 0.7 Hz, 1H), 8.44-8.34 (m, 2H), 7.89-7.79 (m, 2H), 2.41 (d, *J=* 0.7 Hz, 3H); ES-MS *m*/*z* 250.1 [M + H⁺].

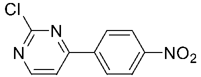

The above formula is 2-chloro-4-(4-nitrophenyl)pyrimidine 2.2: mp: 54-55°C; 54%; ES-MS *m*/*z* 236.1 [M + H⁺].

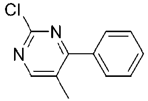

The above formula is 2-chloro-5-methyl-4-phenylpyrimidine 2.3: mp: 123-125°C; 38%; ES-MS *m*/*z* 205.1 [M + H⁺].

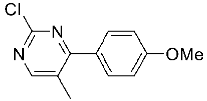

The above formula is 2-chloro-4-(4-methoxyphenyl)-5-methylpyrimidine 2.4: mp: 93-94°C; 94%; ES-MS *m*/*z* 235.1 [M + H⁺].

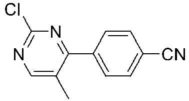

The above formula is 4-(2-chloro-5-methylpyrimidin-4-yl)benzonitrile 2.5: mp: 152-154°C; 83%; ES-MS *m*/*z* 230.0 [M + H⁺].

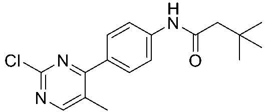

The above formula is N-(4-(2-chloro-5-methylpyrimidine-4-yl)phenyl)-3,3-dimethylbutanamide 2.6: mp: 159-161°C; 72%; ¹H NMR (300 MHz, CDCl₃) δ 8.48 (s, 1H), *7.66 (d, J=* 1.1 Hz, 4H), 2.40 (s, 3H), 2.27 (s, 2H), 1.12 (s, 9H); ES-MS *m*/*z* 318.0 [M + H⁺].

The synthesis process for compound 3 is as follows. Compound 2 (11 mmol) was added in a 150 mL thick-walled pressure-resistant flask, followed by addition of 50 mL of 7 mol/L ammonia-methanol solution. The temperature was raised to 110°C for reaction. After the complete reaction of the raw materials, the reaction solution was evaporated to dryness. 100 mL of water was added, and an aqueous phase was extracted with ethyl acetate (3 × 50 mL). Organic phases were combined. The combined organic phase was washed twice with a saturated sodium chloride solution, and dried with anhydrous sodium sulfate, and a solvent was evaporated to dryness. After column chromatography separation [V (petroleum ether):V (ethyl acetate)=2:1-1:1), target product 3 was obtained.

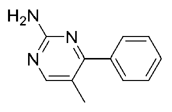

The above formula is 2-amino-5-methyl-4-phenylpyrimidine 3.1: mp: 174-178°C; 65%; ES-MS *m*/*z* 186.1 [M + H⁺].

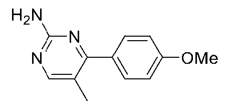

The above formula is 4-(4-methoxyphenyl)-5-methylpyrimidine-2-amine 3.2: mp: 136-138°C; 65%; ¹H NMR (300 MHz, CDCl₃) δ 8.16 (s, 1H), 7.65-7.43 (m, 2H), 7.02-6.93 (m, 2H), 5.03 (s, 2H), 3.86 (s, 3H), 2.22 (s, 3H); ES-MS m/z 216.1 [M + H⁺].

Synthetic route I for compound 4 is as follows.

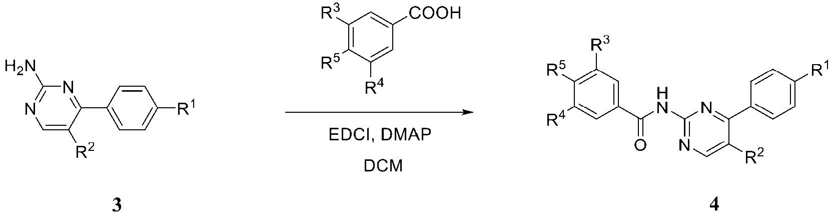

The process for the above-mentioned synthetic route I is as follows. Compound 3 (14 mmol) was weighed into a 250 mL round-bottom flask, followed by addition of 70 mL dichloromethane to dissolve compound 3. Subsequently, benzoic acid (17.5 mmol), 1-ethyl-3-(3-dimethylaminopropyl)carbodiimide hydrochloride (EDCI, 4.026 g, 21 mmol), and 4-dimethylaminopyridine (DMAP, 855 mg, 7 mmol) were added sequentially. The mixture was stirred at room temperature for 96 hours. After the reaction ended, 350 mL of water was added, and an aqueous phase was extracted with dichloromethane (3 × 175 mL). Organic phases were combined. The combined organic phase was washed twice with a saturated sodium chloride solution, and dried with anhydrous sodium sulfate, and a solvent was evaporated to dryness. After column chromatography separation [V (petroleum ether):V (ethyl acetate)=4:1-1:1), target product 4 was obtained.

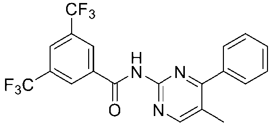

The above formula is N-(5-methyl-4-phenylpyrimidine-2-yl)-3,5-di(trifluoromethyl)benzamide 4.1: mp: 158-159°C; 53%; ¹H NMR (300 MHz, CDCl₃) δ 8.98 (s, 1H), 8.60 (s, 1H), 8.35 (s, 2H), 8.05 (s, 1H), 7.65-7.53 (m, 2H), 7.52-7.42 (m, 3H), 2.38 (s, 3H); ¹³C NMR (75 MHz, CDCl₃) δ 166.5, 163.1, 160.3, 155.7, 137.2, 136.7, 132.5, 132.1, 129.9, 128.8, 128.6, 128.2, 125.5, 124.8, 124.4, 121.2, 16.7; ES-MS m/z 426.1 [M + H⁺].

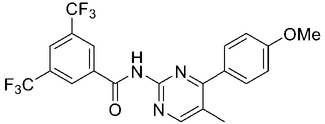

The above formula is N-(4-(4-methoxyphenyl)-5-methylpyrimidine-2-yl)-3,5-bis(trifluoromethyl)benzamide 4.2: mp: 141-142°C; 64%; ¹H NMR (300 MHz, CDCl₃) δ 9.02 (s, 1H), 8.55 (s, 1H), 8.35 (s, 2H), 8.05 (s, 1H), 7.58 (d, *J =* 8.7 Hz, 2H), 6.98 (d, *J =* 8.7 Hz, 2H), 3.87 (s, 3H), 2.40 (s, 3H); ¹³C NMR (75 MHz, CDCl₃) δ 165.8, 163.2, 161.1, 160.2, 155.7, 136.9, 132.5, 132.0, 130.6, 129.5, 128.1, 125.5, 124.8, 123.9, 121.2, 114.0, 55.5, 17.1; ES-MS *m*/*z* 456.1 [M + H⁺].

Synthetic route II for compound 4 is as follows.

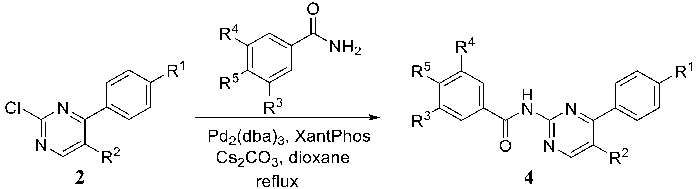

The process for the above-mentioned synthetic route II is as follows. Benzamide (10 mmol), compound 2 (12 mmol), Pd₂(dba)₃ (458 mg, 0.5 mmol), Xantphos (868 mg, 1.5 mmol), and Cs₂CO₃ (4.561 g, 14 mmol) were weighed into a 250 mL three-necked flask. The flask was placed under a positive pressure of nitrogen, and subjected to three vacuumized nitrogen replacement cycles under high vacuum. Under the positive pressure of nitrogen, 1,4-dioxane (100 mL) was added, and the mixture was reacted under reflux overnight. After the reaction ended, 350 mL of water was added, and an aqueous phase was extracted with ethyl acetate (3 × 175 mL). Organic phases were combined. The combined organic phase was washed twice with a saturated sodium chloride solution, and dried with anhydrous sodium sulfate, and a solvent was evaporated to dryness. After column chromatography separation [V (petroleum ether):V (ethyl acetate)=2:1-1:1], target product 4 was obtained.

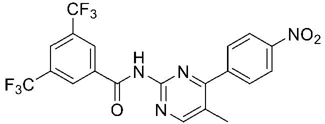

The above formula is N-(5-methyl-4 -(4-nitrophenyl)pyrimidine-2-yl)-3,5-di(trifluoromethyl)benzamide 4.3: mp: 191-192°C; 83%; ¹H NMR (300 MHz, CDCl₃) δ 8.81 (s, 1H), 8.67 (s, 1H), 8.37-8.34 (m, 4H), 8.09 (s, 1H), 7.82 (d, *J =* 9.0 Hz, 2H), 2.41 (s, 3H); ¹³C NMR (75 MHz, CDCl₃) δ 164.0, 163.3, 161.0, 155.9, 148.5, 143.3, 136.5, 132.6, 132.2, 130.1, 128.2, 125.8, 124.7, 124.4, 123.8, 121.1, 16.6; ES-MS m/z 471.1 [M + H⁺].

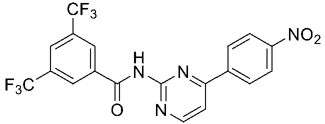

The above formula is N-(4-(4-nitrophenyl)pyrimidine-2-yl)-3,5-bis(trifluoromethyl)benzamide 4.4: mp: 181-183°C; 76%; ¹H NMR (300 MHz, CDCl₃) δ 8.95 (s, 1H), 8.84 (d, *J =* 5.2 Hz, 1H), 8.40 (s, 2H), 8.39-8.33 (m, 2H), 8.30-8.22 (m, 2H), 8.11 (s, 1H), 7.61 (d, *J =* 5.2Hz, 1H); ¹³C NMR (75 MHz, CDCl₃) δ 163.3, 160.0, 157.9, 149.8, 141.6, 136.6, 132.9, 132.4, 128.4, 128.2, 126.0, 124.7, 124.3, 121.1, 113.6; ES-MS *m*/*z* 457.0 [M + H⁺].

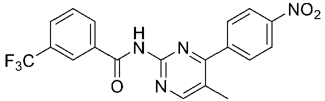

The above formula is N-(5-methyl-4 -(4-nitrophenyl)pyrimidine-2-yl)-3-(trifluoromethyl)benzamide 4.5: mp: 195-196°C; 75%; ¹H NMR (300 MHz, CDCl₃) δ 8.72 (s, 1H), 8.66 (s, 1H), 8.36 (d, *J =* 8.5 Hz, 2H), 8.19 (s, 1H), 8.14 (d, *J =* 7.9 Hz, 1H), 7.89-7.79 (m, 3H), 7.66 (t, *J =* 7.9 Hz, 1H), 2.40 (s, 3H); ¹³C NMR (75 MHz, CDCl₃) δ 164.0, 163.9, 161.09, 161.07, 156.1, 148.5, 143.5, 135.2, 131.7, 131.5, 131.3, 131.0, 130.1, 129.6, 129.1, 125.5, 124.6, 124.1, 123.8, 121.9, 16.6; ES-MS *m*/*z* 403.1 [M + H⁺].

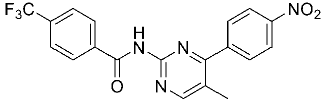

The above formula is N-(5-methyl-4 -(4-nitrophenyl)pyrimidine-2-yl)-4-(trifluoromethyl)benzamide 4.6: mp: 187-188°C; 83%; ¹H NMR (300 MHz, CDCl₃) δ 8.70 (s, 1H), 8.65 (s, 1H), 8.39-8.32 (m, 2H), 8.06 (d, *J=* 8.5 Hz, 2H), 7.86-7.79 (m, 2H), 7.77 (d, *J =* 8.6 Hz, 2H), 2.39 (s, 3H); ¹³C NMR (75 MHz, CDCl₃) δ 164.1, 163.9, 161.1, 156.0, 148.5, 143.5, 137.7, 134.4, 133.9, 130.1, 128.1, 126.0, 124.1, 123.8, 121.8, 16.6; ES-MS *m*/*z* 403.1 [M + H⁺].

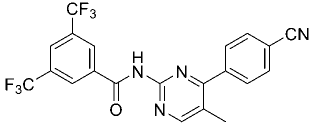

The above formula is N-(4-(4-cyanophenyl)-5-methylpyrimidine-2-yl)-3,5-bis(trifluoromethyl)benzamide 4.7: mp: 214-216°C; 69%; ¹H NMR (300 MHz, CDCl₃) δ 8.91 (s, 1H), 8.65 (s, 1H), 8.37 (s, 2H), 8.08 (s, 1H), 7.87-7.77 (m, 2H), 7.78-7.69 (m, 2H), 2.39 (s, 3H); ¹³C NMR (75 MHz, CDCl₃) δ 164.2, 163.2, 161.0, 155.8, 141.6, 136.6, 132.7, 132.4, 132.2, 129.7, 128.2, 125.8, 124.7, 124.4, 121.1, 118.3, 113.7, 16.6; ES-MS *m*/*z* 451.1 [M + H⁺].

The synthesis process for compound 5 is as follows. Compound 4 (3 mmol), iron powder (672 mg, 12 mmol), ammonium chloride (481 mg, 9 mmol), ethanol (30 mL), and water (7.5 mL) were weighted and sequentially added to a 100 mL round-bottom flask. The mixture was stirred and heated under reflux. After 4 hours, the reaction was monitored. After the raw materials were reacted completely, the reaction solution was filtered with diatomite, and the filter cake was rinsed with 400 mL of ethanol. The filtrate was evaporated to dryness. 300 mL of water was added, and an aqueous phase was extracted with ethyl acetate (3 × 150 mL). Organic phases were combined. The combined organic phase was washed twice with a saturated sodium chloride solution, and dried with anhydrous sodium sulfate, and a solvent was evaporated to dryness. After column chromatography separation [V (petroleum ether):V (ethyl acetate)=2:1], target product 5 was obtained.

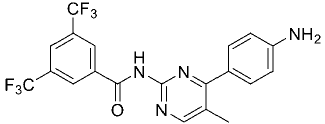

The above formula is N-(4-(4-aminophenyl)-5-methylpyrimidine-2-yl)-3,5-di(trifluoromethyl)benzamide 5.1 (also known as XLL-5471): mp: 108-110°C; 75%; ¹H NMR (300 MHz, CDCl₃) δ 9.28 (s, 1H), 8.51 (s, 1H), 8.30 (s, 2H), 8.02 (s, 1H), 7.42 (d, *J =* 8.5 Hz, 2H), 6.68 (d, *J =* 8.5 Hz, 2H), 3.95 (s, 2H), 2.39 (s, 3H); ¹³C NMR (75 MHz, CDCl₃) δ 165.9, 163.2, 160.2, 155.8, 148.4, 136.9, 132.3, 131.8, 130.6, 128.1, 126.6, 125.3, 124.8, 123.6, 121.2, 114.4, 17.2; ES-MS *m*/*z* 441.1 [M + H⁺].

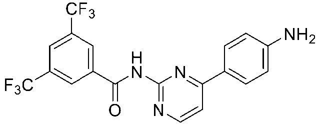

The above formula is N-(4-(4-aminophenyl)pyrimidine-2-yl)-3,5-bis(trifluoromethyl)benzamide 5.2: mp: 145-146°C; 67%; ¹H NMR (300 MHz, CDCl₃) δ 8.85 (s, 1H), 8.59 (s, 1H), 8.37 (s, 2H), 8.07 (m, 2H), 7.87 (d, *J =* 8.6 Hz, 2H), 7.38-7.79 (m, 2H), 6.72 (d, *J =* 8.6 Hz, 2H), 4.06 (s, 2H); ¹³C NMR (75 MHz, CDCl₃) δ 167.3, 165.5, 158.3, 157.5, 150.1, 137.2, 132.5, 132.2, 132.1, 129.0, 128.2, 125.4, 124.8, 121.2, 114.9, 111.5; ES-MS *m*/*z* 427.1 [M + H⁺].

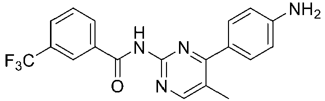

The above formula is N-(4-(4-aminophenyl)-5-methylpyrimidine-2-yl)-3-(trifluoromethyl)benzamide 5.3: mp: 114-116°C; 61%; ¹H NMR (300 MHz, CDCl₃) δ 8.71 (s, 1H), 8.51 (s, 1H), 8.18 (s, 1H), 8.11 (d, *J =* 7.2 Hz, 1H), 7.82 (d, *J* = 7.2 Hz, 1H), 7.62 (t, *J =* 7.2 Hz, 1H), 7.53 (d, *J* = 8.2 Hz, 2H), 6.75 (d, *J =* 8.2 Hz, 2H), 3.92 (s, 2H), 2.40 (s, 3H); ¹³C NMR (75 MHz, CDCl₃) δ 165.9, 164.2, 160.0, 155.8, 148.3, 135.6, 131.5, 130.9, 130.7, 129.4, 128.6, 127.1, 124.7, 123.3, 114.5, 17.3; ES-MS *m*/*z* 373.1 [M + H⁺].

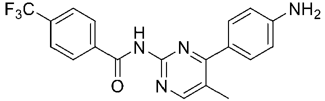

The above formula is N-(4-(4-aminophenyl)-5-methylpyrimidine-2-yl)-4-(trifluoromethyl)benzamide 5.4: mp: 161-163°C; 72%; ¹H NMR (300 MHz, CDCl₃) δ 8.50 (s, 1H), 8.02 (d, *J =* 8.6 Hz, 2H), 7.74 (d, *J =* 8.6 Hz, 2H), 7.52 (d, *J =* 8.5 Hz, 2H), 6.74 (d, *J =* 8.4 Hz, 2H), 2.40 (s, 3H); ¹³C NMR (75 MHz, CDCl₃) δ 165.9, 164.4, 160.1, 155.8, 148.4, 138.2, 133.4, 130.7, 128.1, 127.1, 125.8, 123.3, 121.9, 114.5, 17.3; ES-MS m/z 373.1 [M + H⁺].

(B) The synthetic route for bis(aromatic amide)-substituted pyrimidine biphenyl compound 6 is as follows.

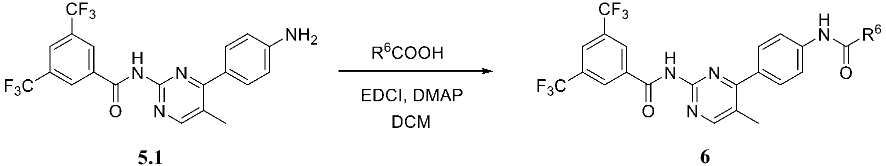

The synthesis process for compound 6 is as follows. Compound 5.1 (0.3 mmol) was weighed into a 50 mL round-bottom flask, followed by addition of 5 mL dichloromethane to dissolve compound 5.1. Subsequently, R⁶COOH (0.375 mmol), EDCI (83 mg, 0.45 mmol), and DMAP (18 mg, 0.15 mmol) were added sequentially. The mixture was stirred at room temperature for 9 hours. After the reaction ended, 10 mL of water was added, and an aqueous phase was extracted with dichloromethane (3 × 5 mL). Organic phases were combined. The combined organic phase was washed twice with a saturated sodium chloride solution, and dried with anhydrous sodium sulfate, and a solvent was evaporated to dryness. After column chromatography separation [V (petroleum ether):V (ethyl acetate)=2:1-1:1), target product 6 was obtained.

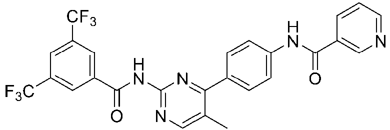

The above formula is N-(4-(2-(3,5-bis(trifluoromethyl)benzamido)-5-methylpyrimidin-4-yl)phenyl)nicotinamide 6.1: mp: 231-232°C; 75%; ¹H NMR (300 MHz, CDCl₃) δ 9.38 (s, 1H), 9.14 (s, 1H), 8.68 (d, *J* = 4.6 Hz, 1H), 8.59 (s, 1H), 8.53 (s, 1H), 8.38 (s, 2H), 8.22 (d, *J =* 8.4 Hz, 1H), 8.06 (s, 1H), 7.74 (d, *J =* 8.4 Hz, 2H), 7.57 (d, *J=* 8.3 Hz, 2H), 7.47-7.36 (m, 1H), 2.38 (s, 3H); ¹³C NMR (75 MHz, DMSO-*d*₆) δ 164.3, 162.8, 160.2, 155.9, 152.2, 148.7, 140.1, 136.7, 135.5, 132.6, 130.6, 130.5, 130.2, 129.7, 129.0, 125.3, 124.9, 123.8, 123.5, 121.3, 119.6, 16.5; ES-MS *m*/*z* 546.1 [M + H⁺].

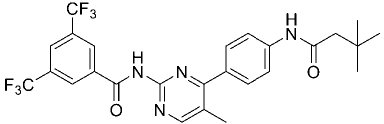

The above formula is N-(4-(4-(3,3-dimethylbutanamido)phenyl)-5-methylpyrimidin-2-yl)-3,5-bis(trifluoromethyl)benzamide 6.2: mp: 121-122°C; 70%; ¹H NMR (300 MHz, CDCl₃) δ 9.04 (s, 1H), 8.56 (s, 1H), 8.38 (s, 2H), 8.06 (s, 1H), 7.65 (d, *J =* 8.4 Hz, 2H), 7.59 (d, *J =* 8.6 Hz, 2H), 7.28 (s, 1H), 2.39 (s, 3H), 2.26 (s, 2H), 1.12 (s, 9H); ¹³C NMR (75 MHz, CDCl₃) δ 170.6, 165.6, 163.1, 160.3, 160.1, 155.7, 148.4, 139.6, 136.9, 136.7, 132.4, 132.0, 130.6, 129.8, 128.1, 126.7, 125.5, 124.8, 124.2, 123.7, 121.2, 119.5, 114.4, 51.7, 31.5, 29.9, 17.3, 16.9; ES-MS *m*/*z* 539.2 [M + H⁺].

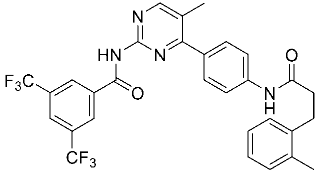

The above formula is N-(5-methyl-4-(4-(3-(o-tolyl)propanamido)phenyl)pyrimidin-2-yl)-3,5-bis(trifluoromethyl)benzamide 6.3: mp: 107-109°C; 61%; ¹H NMR (300 MHz, CDCl₃) δ 9.43 (s, 1H), 8.52 (s, 1H), 8.40 (s, 2H), 8.04 (s, 1H), 7.73-7.44 (m, 4H), 7.13 (s, 4H), 3.03 (s, 2H), 2.62 (s, 2H), 2.35 (s, 3H), 2.31 (s, 3H); ES-MS *m*/*z* 587.0 [M + H⁺].

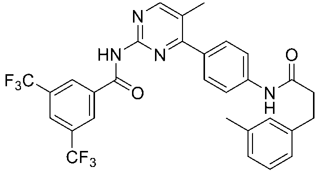

The above formula is N-(5-methyl-4-(4-(3-(m-tolyl)propanamido)phenyl)pyrimidin-2-yl)-3,5-bis(trifluoromethyl)benzamide 6.4: mp: 108-110°C; 75%; ¹H NMR (300 MHz, CDCl₃) δ 9.05 (s, 1H), 8.55 (s, 1H), 8.38 (s, 2H), 8.06 (s, 1H), 7.56 (s, 4H), 7.31 (s, 1H), 7.19 (t, *J =* 7.7 Hz, 1H), 7.05 (s, 2H), 7.03 (s, 1H), 3.02 (t, *J =* 7.4 Hz, 2H), 2.68 (t, *J* = 7.5 Hz, 2H), 2.38 (s, 3H), 2.32 (s, 3H); ES-MS *m*/*z* 587.0 [M + H⁺].

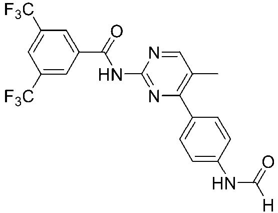

The above formula is N-(4-(4-formamidophenyl)-5-methylpyrimidin-2-yl)-3,5-bis(trifluoromethyl)benzamide 6.5: mp: 237-239°C; 70%; ¹H NMR (300 MHz, CDCl₃) δ 10.19 (s, 1H), 8.99 (d, *J* = 11.1 Hz, 1H), 8.59 (s, 1H), 8.52 (s, 2H), 8.34 (s, 1H), 7.93 (s, 1H), 7.46 (d, *J =* 8.4 Hz, 2H), 6.86 (d, *J =* 8.4 Hz, 2H), 2.33 (s, 3H); ES-MS *m*/*z* 469.0 [M + H⁺].

(C) The synthetic route for amine-substituted pyrimidine biphenyl compound 8 is as follows.

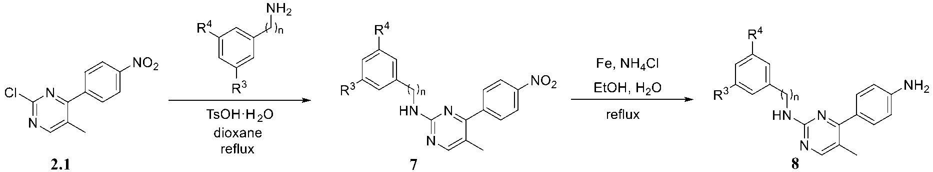

The synthesis process for compound 7 is as follows. Compound 2.1 (249 mg, 1 mmol) was weighed into a 50 mL round-bottom flask, followed by addition of 10 mL of 1,4-dioxane for dissolving compound 2.1. Subsequently, TsOH·H₂O (304 mg, 1.6 mmol) and amine (4 mmol) were added sequentially. The mixture was stirred under reflux to initiate the reaction. After there were no raw materials detected by TLC, the reaction was stopped. A solvent was evaporated to dryness, and 30 mL of water was added. Extraction was performed with dichloromethane (3 × 20 mL), and organic phases were combined. The combined organic phase was dried with anhydrous sodium sulfate, and the organic phase was evaporated to dryness under reduced pressure. After column chromatography separation [V (petroleum ether):V (ethyl acetate):V (acetone) = 5:1:1], target product 7 was obtained.

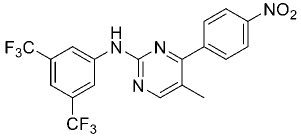

The above formula is N-(3,5-bis(trifluoromethyl)phenyl)-5-methyl-4-(4-nitrophenyl)pyrimidin-2-amine 7.1: mp: 180-182°C; 68%; ¹H NMR (300 MHz, CDCl₃) δ 8.48 (s, 1H), 8.42-8.34 (m, 2H), 8.21 (s, 2H), 7.90-7.82 (m, 2H), 7.57 (s, 1H), 7.51 (s, 1H), 2.36 (s, 3H); ES-MS *m*/*z* 443.1 [M + H⁺].

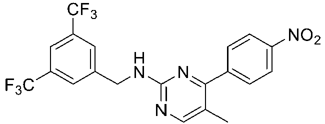

The above formula is N-(3,5-bis(trifluoromethyl)benzyl)-5-methyl-4-(4-nitrophenyl)pyrimidin-2-amine 7.2: mp: 158-160°C; 52%; ¹H NMR (300 MHz, CDCl₃) δ 8.35-8.28 (m, 2H), 8.26 (s, 1H), 7.84 (s, 2H), 7.78 (s, 1H), 7.73-7.65 (m, 2H), 4.76 (d, *J =* 6.3Hz, 2H), 2.12 (s, 3H); ES-MS m/z 457.1 [M + H⁺].

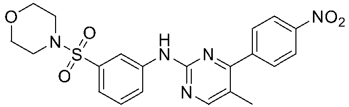

The above compound is 5-methyl-N-(3-(morpholine sulfonyl)phenyl)-4-(4-nitrophenyl)pyrimidin-2-amine 7.3: mp: 196-198°C; 90%; ¹H NMR (300 MHz, DMSO-*d*₆) δ 10.12 (s, 1H), 8.57 (s, 1H), 8.44 (t, *J =* 1.9 Hz, 1H), 8.41-8.32 (m, 2H), 8.02-7.91 (m, 3H), 7.55 (t, *J =* 8.0 Hz, 1H), 7.29-7.23 (m, 1H), 3.62-3.54 (m, 4H), 2.87-2.79 (m, 4H), 2.26 (s, 3H); ¹³C NMR (75 MHz, CDCl₃) δ 162.3, 160.5, 158.0, 147.6, 144.0, 141.4, 134.6, 130.0, 129.4,123.2, 122.5, 119.1, 116.6, 65.1, 45.8, 15.4; ES-MS *m*/*z* 456.1 [M + H⁺].

The synthesis process for compound 8 is as follows. Compound 7 (3 mmol), iron powder (672 mg, 12 mmol), ammonium chloride (481 mg, 9 mmol), ethanol (30 mL), and water (7.5 mL) were weighted and sequentially added to a 100 mL round-bottom flask. The mixture was stirred and heated under reflux. The reaction was monitored by TCL. After the raw materials were reacted completely, the reaction solution was filtered with diatomite, and the filter cake was rinsed with 400 mL of ethanol. The filtrate was evaporated to dryness. 300 mL of water was added, and an aqueous phase was extracted with ethyl acetate (3 × 150 mL). Organic phases were combined, and the combined organic phase was washed twice with a saturated sodium chloride solution, and dried with anhydrous sodium sulfate, and a solvent was evaporated to dryness. After column chromatography separation [V (petroleum ether):V (ethyl acetate) = 2:1], target product 8 was obtained.

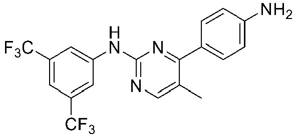

The above formula is 4-(4-aminophenyl)-N-(3,5-bis(trifluoromethyl)phenyl)-5-methylpyrimidin-2-amine 8.1: mp: 189-190°C; 80%; ¹H NMR (300 MHz, CDCl₃) δ 8.31 (s, 1H), 8.25 (s, 2H), 7.77 (s, 1H), 7.63 (d, *J =* 8.7 Hz, 2H), 7.47 (s, 1H), 6.78 (d, *J =* 8.7 Hz, 2H), 2.39 (s, 3H); ¹³C NMR (75 MHz, CDCl₃) δ 165.3, 159.9, 157.9, 148.3, 141.9, 132.3, 131.8, 130.8, 127.8, 125.4, 121.8, 119.7, 118.1, 114.5, 17.3; ES-MS *m*/*z* 413.1 [M + H⁺].

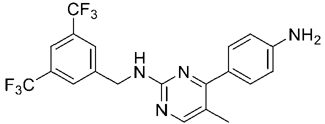

The above formula is 4-(4-aminophenyl)-N-(3,5-bis(trifluoromethyl)benzyl)-5-methylpyrimidin-2-amine 8.2: mp: 132-134°C; 86%; ¹H NMR (300 MHz, CDCl₃) δ 8.11 (s, 1H), 7.83 (s, 2H), 7.75 (s, 1H), 7.41 (d, J = 8.6 Hz, 2H), 6.70 (d, J = 8.6 Hz, 2H), 5.83 (s, 1H), 4.71 (d, J = 6.2 Hz, 2H), 2.24 (s, 3H); ¹³C NMR (75 MHz, CDCl₃) δ 165.6, 160.9, 159.8, 147.7, 143.3, 131.8, 131.3, 130.4, 128.6, 127.7, 125.4, 121.7, 120.9, 117.1, 114.4, 44.8, 16.8; ES-MS *m*/*z* 427.1 [M + H⁺].

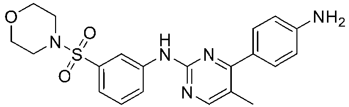

The above formula is 4-(4-aminophenyl)-5-methyl-N-(3-(morpholine sulfonyl)phenyl)pyrimidin-2-amine 8.3: mp: 196-198°C; 81%; ¹H NMR (300 MHz, DMSO-*d*₆) δ 9.83 (s, 1H), 8.61 (s, 1H), 8.34 (s, 1H), 7.94-7.80 (m, 1H), 7.64-7.46(m, 3H), 7.32-7.13(m, 1H),6.66 (d, *J =* 8.6 Hz, 2H), 5.58 (s, 2H), 3.66-3.53 (m, 4H), 2.90-2.82 (m, 4H), 2.31 (s, 3H); ES-MS *m*/*z* 426.2 [M + H⁺]. ¹³C NMR (75 MHz, DMSO-*d*₆) 164.0, 159.5, 158.0, 150.1, 142.0, 134.6, 130.2, 129.2, 124.7, 122.2, 119.1, 117.8, 116.1, 112.8, 65.1, 45.8, 16.8; ES-MS *m*/*z* 426.4 [M + H⁺].

(D) The synthetic route for N-(4-(4-aminophenyl)-5-methylpyrimidin-2-yl)-N-methyl-3,5-bis(trifluoromethyl)benzamide 10 is as follows.

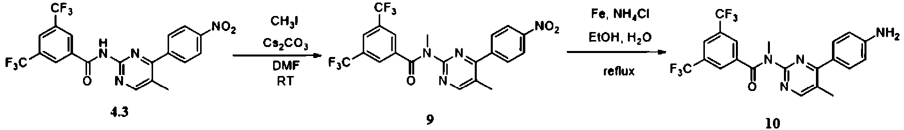

The synthesis process for compound 9 is as follows. Compound 4.3 (235mg, 0.5 mmol) was weighed into a 50 mL round-bottom flask, followed by addition of 5.5 mL N,N-dimethylformamide to dissolve compound 4.3. Subsequently, iodomethane (142 mg, 1 mmol), and cesium carbonate (163 mg, 0.5 mmol) were added sequentially. The mixture was stirred at room temperature. TLC monitoring was performed. After the raw materials were completely reacted, the reaction was ended. 15 mL of water was added, and an aqueous phase was extracted with ethyl acetate (3 × 7.5 mL). Organic phases were combined, and the combined organic phase was washed twice with a saturated sodium chloride solution, and dried with anhydrous sodium sulfate, and a solvent was evaporated to dryness. After column chromatography separation [V (petroleum ether):V (ethyl acetate) = 1:1], target product 9 was obtained.

N-methyl-N-(5-methyl-4-(4-nitrophenyl)pyrimidin-2-yl)-3,5-bis(trifluoromethyl)benzamide 9 (compound 9): mp: 109-111°C; 87%; ¹H NMR (300 MHz, CDCl₃) δ 8.48 (s, 1H), 8.25-8.19 (m, 2H), 7.82 (s, 1H), 7.80 (s, 2H), 7.29-7.22 (m, 2H), 3.74 (s, 3H), 2.32 (s, 3H); ES-MS *m*/*z* 485.1 [M + H⁺].

The synthesis process for compound 10 is as follows. Compound 9 (1.452g, 3 mmol), iron powder (672 mg, 12 mmol), ammonium chloride (481 mg, 9 mmol), ethanol (30 mL), and water (7.5 mL) were weighted and sequentially added to a 100 mL round-bottom flask. The mixture was stirred and under reflux. The reaction was monitored after 4 hours. After the raw materials were reacted completely, the reaction mixture was filtered with diatomite, and the filter cake was rinsed with 400 mL of ethanol. The filtrate was evaporated to dryness. 300 mL of water was added, and an aqueous phase was extracted with ethyl acetate (3 × 150 mL). Organic phases were combined, and the combined organic phase was washed twice with a saturated sodium chloride solution, and dried with anhydrous sodium sulfate, and a solvent was evaporated to dryness. After column chromatography separation [V (petroleum ether):V (ethyl acetate) = 2:1], target product 10 was obtained.

N-(4-(4-aminophenyl)-5-methylpyrimidin-2-yl)-N-methyl-3,5-bis(trifluoromethyl)benzamide 10: mp: 106-107°C; 71%; ¹H NMR (300 MHz, CDCl₃) δ 8.33 (s, 1H), 7.80 (s, 2H), 6.95-6.87 (m, 2H), 6.62-6.54 (m, 2H), 3.71 (s, 3H), 2.34 (s, 3H); ¹³C NMR (75 MHz, CDCl₃) δ 169.1, 164.8, 159.9, 159.4, 148.5, 140.4, 131.8, 131.4, 130.4, 128.1, 126.5, 124.9, 123.6, 123.0, 114.2, 34.5, 17.3; ES-MS *m*/*z* 455.1 [M + H⁺].

(E) The synthetic route for 4-(4-aminophenyl)-5-methylpyrimidin-2-amine 11 is as follows.

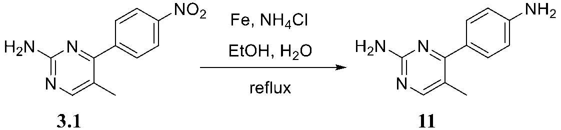

The synthesis process for compound 11 is as follows. Compound 3.1 (690 mg, 3 mmol), iron powder (672 mg, 12 mmol), ammonium chloride (481 mg, 9 mmol), ethanol (30 mL), and water (7.5 mL) were weighted and sequentially added to a 100 mL round-bottom flask. The mixture was stirred and under reflux. After 4 hours, the reaction was monitored. After the raw materials were reacted completely, the reaction solution was filtered with diatomite, and the filter cake was rinsed with 400 mL of ethanol. The filtrate was evaporated to dryness. 300 mL of water was added, and an aqueous phase was extracted with ethyl acetate (3 × 150 mL). Organic phases were combined, and the combined organic phase was washed twice with a saturated sodium chloride solution, and dried with anhydrous sodium sulfate, and a solvent was evaporated to dryness. After column chromatography separation [V (petroleum ether):V (ethyl acetate) = 2:1], target product 11 was obtained.

4-(4-aminophenyl)-5-methylpyrimidin-2-amine 11: mp: 188-190°C; 72 %; ¹H NMR (300 MHz, CDCl₃) δ 8.12 (s, 1H), 7.54-7.37 (m, 2H), 6.79-6.59 (m, 2H), 5.04 (s, 2H), 3.50 (s, 2H), 2.22 (s, 3H); ES-MS *m*/*z* 201.1 [M + H⁺].

(F) The synthetic route for N-(4-(2-((3-(N-benzylsulfamoyl)phenyl)amino)-5-methylpyrimidin-4-yl)phenyl)-3,3-dimethylbutanamide 12 is as follows.

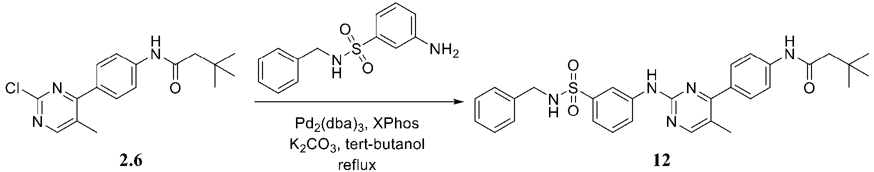

The synthesis process for compound 12 is as follows. N-benzyl-3-aminobenzenesulfonamide (236 mg, 0.9 mmol), compound 2.6 (300 mg, 0.945 mmol), Pd₂(dba)₃ (82 mg, 0.09 mmol), XPhos (43 mg, 0.09 mmol), and K₂CO₃ (373 mg, 2.7 mmol) were weighed into a 25 mL three-necked flask. The flask was placed under the positive pressure of nitrogen, and subjected to three vacuumized nitrogen replacement cycles under high vacuum. Under the positive pressure of nitrogen, tert-butanol (9 mL) was added, and reaction was performed under reflux overnight. After the reaction ended, 50 mL of water was added, and an aqueous phase was extracted with ethyl acetate (3 × 25 mL). Organic phases were combined, and the combined organic phase was washed twice with a saturated sodium chloride solution, and dried with anhydrous sodium sulfate, and a solvent was evaporated to dryness. After column chromatography separation [V (petroleum ether):V (ethyl acetate) = 100:1], target product 12 was obtained.

N-(4-(2-((3-(N-benzylsulfamoyl)phenyl)amino)-5-methylpyrimidin-4-yl)phenyl)-3,3-dimethylbutanamide 12: mp: 225-226°C; 60%; ¹H NMR (300 MHz, DMSO-*d*₆) δ 10.02 (s, 1H), 9.91 (s, 1H), 8.59 (t, *J =* 1.8 Hz, 1H), 8.45 (s, 1H), 8.07 (t, *J =* 6.4 Hz, 1H), 7.91-7.86 (m, 1H), 7.76 (s, 4H), 7.47 (t, *J =* 8.0 Hz, 1H), 7.38-7.31 (m, 1H), 7.29-7.19 (m, 5H), 3.99 (d, *J =* 6.3 Hz, 2H), 2.30 (s, 3H), 2.23 (s, 2H), 1.04 (s, 9H); ¹³C NMR (75 MHz, DMSO-*d*₆) δ 170.3, 163.7, 163.5, 160.1, 158.3, 141.6, 141.1, 140.3, 137.8, 132.4, 129.6, 129.3, 128.2, 127.5, 127.1, 121.5, 118.5, 118.3, 115.9, 49.6, 46.2, 30.9, 29.6, 16.3; ES-MS *m*/*z* 544.2 [M + H⁺].

### Embodiment 11: analysis of the effect of compounds on enhancing the susceptibility of tumor cells to NK cell-induced killing

Experimental method: 5000 NCI-H446 cells were inoculated into a 96-well plate, and 5 µM of the following compounds were added, respectively, for culturing overnight. Subsequently, human primary NK cells were added (at an E/T ratio of 5:1) for co-incubation for 16 hours. The killing activity of the NK cells against NCI-H446 cells in different treatment groups was detected using a calcein release experiment.

The specific experiments are as follows.

The following compounds have the effect of enhancing the susceptibility of tumor cells to NK cell-induced killing, with a killing ratio against NCI-H446 cells greater than that of the negative control: compound 4.1, compound 4.2, compound 4.3, compound 4.7, compound 5.1, compound 5.3, compound 5.4, compound 6.2, compound 6.3, compound 6.4, compound 6.5, compound 8.1, compound 8.3, and compound 12.

The following compounds have no the effect of enhancing the susceptibility of tumor cells to NK cell-induced killing, with a killing ratio against NCI-H446 cells less than that of the negative control: compound 5.2, compound 6.1, compound 8.2, compound 10, and compound 11.

## Claims

1. A compound, a pharmaceutically acceptable salt, a solvent compound, or a deuterated compound thereof, a structure being set forth in formula (I), 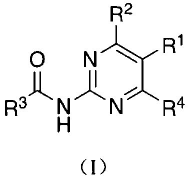 wherein
R¹ is selected from methyl, deuterated methyl, and fluoromethyl,
R² is selected from hydrogen, deuterium, halogen, C1-C3 alkyl, and C1-C3 alkoxy,
R³ and R⁴ are independently selected from cycloalkyl, aryl, and heteroaryl, and the cycloalkyl, the aryl, and the heteroaryl of R³ and R⁴ are optionally substituted with 1, 2, 3, 4, or 5 R, wherein
the R is selected from hydrogen, deuterium, halogen, hydroxyl, amino, trifluoromethyl, cyano, ester, carbonyl, acyl, aminoacyl, amido, sulfonyl, aminosulfonyl, sulfonamido, alkyl, alkoxy, aryl, furyl, thienyl, pyridyl, oxazolyl, and pyrazolyl.

2. The compound, the pharmaceutically acceptable salt, the solvent compound, or the deuterated compound thereof according to claim 1, wherein R² is selected from hydrogen and deuterium.

3. The compound, the pharmaceutically acceptable salt, the solvent compound, or the deuterated compound thereof according to claim 1, wherein R³ and R⁴ are selected from aryl and heteroaryl.

4. An application of the compound, the pharmaceutically acceptable salt, the solvent compound, or the deuterated compound thereof according to any one of claims 1-3 as a CD97 and GSDME interaction inhibitor having anti-tumor activity in the preparation of a drug for treating a cancer.

5. The application according to claim 4, wherein the cancer comprises a malignant tumor with dual positive expression of GSDME and CD97, an amino acid sequence of GSDME is set forth in SEQ ID NO.1, and an amino acid sequence of CD97 is set forth in SEQ ID NO.2.

6. The application according to claim 5, wherein the malignant tumor is lung cancer.

7. The application according to claim 5, wherein the malignant tumor is breast cancer.

8. The application according to claim 5, wherein the malignant tumor is colorectal cancer.

9. The application according to claim 5, wherein the malignant tumor is melanoma.
